# EUROPEAN PATENT SPECIFICATION

(11) **EP 3 452 062 B1**
(45) Date of publication and mention of the grant of the patent: **06.05.2026**
(21) Application number: 17724674.1
(22) Date of filing: 03.05.2017
(51) Int. Cl.: A61P 29/00, A61P 37/02, A61P 37/06, A61P 37/08, A61P 43/00, C12N 5/0781, C12N 5/0783, A61K 40/11, A61K 40/13, A61K 40/22, A61K 40/24, A61K 40/41

(54) **INTRACELLULAR DELIVERY OF BIOMOLECULES TO INDUCE TOLERANCE**
INTRAZELLULÄRE VERABREICHUNG VON BIOMOLEKÜLEN ZUR INDUKTION VON TOLERANZ
ADMINISTRATION INTRACELLULAIRE DE BIOMOLÉCULES POUR INDUIRE UNE TOLÉRANCE

(30) Priority: 03.05.2016 US 201662331384 P
(43) Date of publication of application: 13.03.2019
(73) Proprietor: Stemcell Technologies Canada Inc., Vancouver, British Columbia V6A 1B6 (CA)
(72) Inventor: LOUGHHEAD, Scott, Watertown, MA 02472 (US); GILBERT, Jonathan, B., Watertown, MA 02472 (US); BERNSTEIN, Howard, Watertown, MA 02472 (US); SHAREI, Armon, R., Watertown, MA 02472 (US); MOORE, Finola, Watertown, MA 02472 (US)
(74) Representative: Mathys & Squire
(86) International application number: PCT/US2017/030933
(87) International publication number: WO 2017/192786

(56) References cited:
- WO-A1-03/020039
- WO-A1-2013/059343
- WO-A1-2016/070136
- WO-A1-2017/041050
- WO-A2-02/067863
- US-A1- 2008 311 140
- WEN JING SIM ET AL: "Metabolism Is Central to Tolerogenic Dendritic Cell Function", MEDIATORS OF INFLAMMATION., vol. 2016, 1 January 2016 (2016-01-01), GB, pages 1 - 10, XP055651840, ISSN: 0962-9351, DOI: 10.1155/2016/2636701
- MARTIN P. STEWART ET AL: "In vitro and ex vivo strategies for intracellular delivery", NATURE, vol. 538, no. 7624, 12 October 2016 (2016-10-12), pages 183 - 192, XP055388055, ISSN: 0028-0836, DOI: 10.1038/nature19764
- STEINMAN RALPH M ET AL: "Tolerogenic dendritic cells", ANNUAL REVIEW OF IMMUNOLOGY, ANNUAL REVIEWS INC, US, vol. 21, 1 January 2003 (2003-01-01), pages 685 - 711, XP002433336, ISSN: 0732-0582, DOI: 10.1146/ANNUREV.IMMUNOL.21.120601.141040
- RUTELLA SERGIO ET AL: "Tolerogenic dendritic cells: cytokine modulation comes of age", BLOOD, THE AMERICAN SOCIETY OF HEMATOLOGY, US, vol. 108, no. 5, 1 September 2006 (2006-09-01), pages 1435 - 1440, XP002648261, ISSN: 0006-4971, [retrieved on 20060509], DOI: 10.1182/BLOOD-2006-03-006403

## Description

### FIELD OF THE INVENTION

The present disclosure relates generally to methods for suppressing an immune response or inducing tolerance by delivering an antigen into a tolerogenic cell by passing a cell suspension through a cell-deforming constriction.

### BACKGROUND

Undesired immune responses contribute to autoimmunity, transplant rejection, allergy, and anti-drug responses. Autoimmunity develops when an organism mounts an anti-self response, usually as a result of a dysregulated immune response against self-antigens. Autoimmune diseases include, for example, type I diabetes, systemic lupus erythematosus, rheumatoid arthritis, autoimmune hemolytic anemia, and multiple sclerosis. Pathogenic immune responses after transplantation of a donor organ in a receiving organism can lead to rejection of the transplant and decreased patient survival. In addition, unwanted immune responses against food and environmental antigens drive allergic diseases such as asthma, food allergy, and atopic dermatitis. Therefore, approaches to establish immunological tolerance to an antigen are a focus of intense therapeutic development.

Current intracellular delivery methods are not effective at modulating cell phenotype or function in order to induce antigen-specific tolerance. Thus, there is an unmet need for intracellular delivery techniques that can load antigen and tolerogenic factors into the cytoplasm of cells and drive a powerful immunosuppression response for the treatment of pathogenic immune responses underlying autoimmune diseases and transplant rejection. References that describe methods of using microfluidic constrictions to deliver compounds to cells include WO2013059343, WO2015023982, WO2016070136, WO2016077761, and PCT/US2016/13113.

WO 2016/070136 A1 describes a method and device for preferentially delivering a compound such as an antigen to the cytosol of an immune cell by passing a cell suspension comprising the target immune cell through a microfluidic device and contacting the suspension with the compound(s) or payload to be delivered.

WO 2017/041050 A1 describes methods and devices for delivering a compound into a cell by passing a cell suspension through a surface containing pores, wherein the pores deform the cell thereby causing a perturbation of the cell such that the compound enters the cell, wherein the cell suspension is contacted with the compound.

Stewart MP et al. ("In vitro and ex vivo strategies for intracellular delivery", NATURE, vol. 538, no. 7624, 12 October 2016 (2016-10-12), pages 183-192) describes in vitro and ex vivo intracellular delivery approaches with a focus on membrane-disruption-based delivery methods.

WO 2013/059343 A1 describes a microfluidic system for causing perturbations in a cell membrane that includes a microfluidic channel defining a lumen and a cell-deforming constriction.

WO 03/020039 A1 describes methods for inducing immune tolerance to one or more specific antigens in a host mammal by engineering white blood cells, in vitro, to express an antigen which is not native to the host mammal.

Steinman RM et al. ("Tolerogenic dendritic cells", ANNUAL REVIEW OF IMMUNOLOGY, ANNUAL REVIEWS INC, US, vol. 21, 1 January 2003 (2003-01-01), pages 685-711) describes the role of natural dendritic cells (DCs) in T cell tolerance, covering aspects such as T cell deletion and control of suppressor and regulatory T cells.

Rutella S et al. ("Tolerogenic dendritic cells: cytokine modulation comes of age", BLOOD, vol. 108, no. 5, 1 September 2006 (2006-09-01), pages 1435-1440) describes natural tolerogenic dendritic cells and teaches growth factors that modulate DC maturation and favor differentiation of tolerogenic DCs.

WO 02/067863 A2 describes a method of altering the reactivity of a patient's own defense cells by treating antigen presenting cells (APCs) from the patient so as to make them tolerogenic to specific antigen, and describes incubating APCs with an inhibitory cytokine or an antigen to generate tolerogenic APCs.

US 2008/311140 A1 describes genetically modified dendritic cells expressing at least two immunosuppressive molecules which have the ability to induce tolerance.

Sim WJ et al. ("Metabolism Is Central to Tolerogenic Dendritic Cell Function", MEDIATORS OF INFLAMMATION., vol. 2016, 1 January 2016 (2016-01-01), pages 1-10) describes natural immune tolerance of DCs and teaches the role of metabolic reprogramming in making DCs immunogenic or tolerogenic.

### BRIEF SUMMARY OF THE INVENTION

The present invention provides a method for delivering an antigen into a tolerogenic immune cell or an immunosuppressive immune cell, the method comprising passing a cell suspension comprising the tolerogenic immune cell or the immunosuppressive immune cell through a constriction, wherein said constriction deforms the tolerogenic immune cell or the immunosuppressive immune cell, causing a perturbation of the cell such that the antigen can enter the cell, and contacting said cell suspension with the antigen, wherein the immune cell is a B cell or a T cell, wherein the antigen is: (i) a polypeptide antigen; (ii) a disease-associated antigen; (iii) a foreign antigen; (iv) a self-antigen; (v) a therapeutic agent; (vi) an allograft transplantation antigen; (vii) a non-protein antigen; (viii) an antigen associated with a virus; or (ix) an adenovirus, adeno-associated virus, baculovirus, herpes virus, or retrovirus, wherein an immune response is suppressed by at least about 10% and wherein the suppressed immune response comprises decreased production and/or secretion of one or more inflammatory cytokines, wherein the tolerogenic immune cell or the immunosuppressive immune cell comprises intracellularly a tolerogenic factor comprising a nucleic acid encoding an arginase-1 (ARG1), a nitric-oxide synthase 2 (NOS2), an indoleamine 2,3-dioxygenase (IDO), an IL-4, an IL-10, an IL-13, a transforming growth factor-beta (TGF-β), an IL-35, or an IFN-α, and wherein the tolerogenic immune cell or the immunosuppressive immune cell induces an immunological unresponsiveness or decreased responsiveness to challenge with the antigen.

The present invention provides a method for inducing antigen-specific tolerance in an individual, the method comprising passing a cell suspension comprising a tolerogenic immune cell through a constriction, wherein said constriction deforms the cell thereby causing a perturbation of the cell such that an antigen can enter the immune cell, and introducing the immune cell into the individual, wherein the tolerogenic immune cell is capable of inducing tolerance to the antigen, for example by presentation of said antigen by said tolerogenic immune cell. The immune cell is a B cell or a T cell, wherein the antigen is: (i) a polypeptide antigen; (ii) a disease-associated antigen; (iii) a foreign antigen; (iv) a self-antigen; (v) a therapeutic agent; (vi) an allograft transplantation antigen; (vii) a non-protein antigen; (viii) an antigen associated with a virus; or (ix) an adenovirus, adeno-associated virus, baculovirus, herpes virus, or retrovirus, wherein an immune response is suppressed by at least about 10% and wherein the suppressed immune response comprises decreased production and/or secretion of one or more inflammatory cytokines, wherein the tolerogenic immune cell comprises intracellularly a tolerogenic factor comprising a nucleic acid encoding an arginase-1 (ARG1), a nitric-oxide synthase 2 (NOS2), an indoleamine 2,3-dioxygenase (IDO), an IL-4, an IL-10, an IL-13, a transforming growth factor-beta (TGF-β), an IL-35, or an IFN-α, and wherein the tolerogenic immune cell induces an immunological unresponsiveness or decreased responsiveness to challenge with the antigen

Certain aspects of the present invention provide a method for suppressing an immune response in an individual, the method comprising passing a cell suspension comprising an immunosuppressive immune cell through a constriction, wherein said constriction deforms the cell thereby causing a perturbation of the cell such that an antigen can enter the immune cell, and introducing the immune cell into the individual, wherein presentation of said antigen by said immunosuppressive immune cell suppresses an immune response to the antigen. The immune cell is a B cell or a T cell, wherein the antigen is: (i) a polypeptide antigen; (ii) a disease-associated antigen; (iii) a foreign antigen; (iv) a self-antigen; (v) a therapeutic agent; (vi) an allograft transplantation antigen; (vii) a non-protein antigen; (viii) an antigen associated with a virus; or (ix) an adenovirus, adeno-associated virus, baculovirus, herpes virus, or retrovirus, wherein an immune response is suppressed by at least about 10% and wherein the suppressed immune response comprises decreased production and/or secretion of one or more inflammatory cytokines, wherein the immunosuppressive immune cell comprises intracellularly a tolerogenic factor comprising a nucleic acid encoding an arginase-1 (ARG1), a nitric-oxide synthase 2 (NOS2), an indoleamine 2,3-dioxygenase (IDO), an IL-4, an IL-10, an IL-13, a transforming growth factor-beta (TGF-β), an IL-35, or an IFN-α, and wherein the immunosuppressive immune cell induces an immunological unresponsiveness or decreased responsiveness to challenge with the antigen.

Certain aspects of the present invention provide a method for inducing antigen-specific tolerance in an individual, the method comprising introducing a tolerogenic immune cell or immunosuppressive immune cell into the individual, wherein the immune cell comprises an antigen and a tolerogenic factor, wherein the antigen and the tolerogenic factor were introduced to the immune cell by passing the immune cell through a constriction, wherein said constriction deformed the cell thereby causing a perturbation of the cell such that the antigen and the tolerogenic factor entered the immune cell. The immune cell is a B cell or a T cell, wherein the antigen is: (i) a polypeptide antigen; (ii) a disease-associated antigen; (iii) a foreign antigen; (iv) a self-antigen; (v) a therapeutic agent; (vi) an allograft transplantation antigen; (vii) a non-protein antigen; (viii) an antigen associated with a virus; or (ix) an adenovirus, adeno-associated virus, baculovirus, herpes virus, or retrovirus, wherein an immune response is suppressed by at least about 10% and wherein the suppressed immune response comprises decreased production and/or secretion of one or more inflammatory cytokines, wherein the tolerogenic immune cell or the immunosuppressive immune cell comprises intracellularly a tolerogenic factor comprising a nucleic acid encoding an arginase-1 (ARG1), a nitric-oxide synthase 2 (NOS2), an indoleamine 2,3-dioxygenase (IDO), an IL-4, an IL-10, an IL-13, a transforming growth factor-beta (TGF-β), an IL-35, or an IFN-α, and wherein the tolerogenic immune cell or the immunosuppressive immune cell induces an immunological unresponsiveness or decreased responsiveness to challenge with the antigen.

Certain aspects of the present invention provide a method for suppressing an immune response in an individual, the method comprising introducing a tolerogenic immune cell or immunosuppressive immune cell into the individual, wherein the immune cell comprises an antigen and a tolerogenic factor, wherein the antigen and the tolerogenic factor were introduced to the immune cell by passing the immune cell through a constriction, wherein said constriction deformed the cell thereby causing a perturbation of the cell such that the antigen and the tolerogenic factor entered the immune cell. The immune cell is a B cell or a T cell, wherein the antigen is: (i) a polypeptide antigen; (ii) a disease-associated antigen; (iii) a foreign antigen; (iv) a self-antigen; (v) a therapeutic agent; (vi) an allograft transplantation antigen; (vii) a non-protein antigen; (viii) an antigen associated with a virus; or (ix) an adenovirus, adeno-associated virus, baculovirus, herpes virus, or retrovirus, wherein an immune response is suppressed by at least about 10% and wherein the suppressed immune response comprises decreased production and/or secretion of one or more inflammatory cytokines, wherein the tolerogenic immune cell or the immunosuppressive immune cell comprises intracellularly a tolerogenic factor comprising a nucleic acid encoding an arginase-1 (ARG1), a nitric-oxide synthase 2 (NOS2), an indoleamine 2,3-dioxygenase (IDO), an IL-4, an IL-10, an IL-13, a transforming growth factor-beta (TGF-β), an IL-35, or an IFN-α, and wherein the tolerogenic immune cell or the immunosuppressive immune cell induces an immunological unresponsiveness or decreased responsiveness to challenge with the antigen.

Certain aspects of the present disclosure provide a method for generating a tolerogenic immune cell, the method comprising passing a cell suspension comprising an immune cell through a constriction, wherein said constriction deforms the cell thereby causing a perturbation of the cell such that a tolerogenic factor enters the immune cell, thereby generating a tolerogenic immune cell.

Certain aspects of the present disclosure provide a method for generating an immunosuppressive immune cell, the method comprising passing a cell suspension comprising an immune cell through a constriction, wherein said constriction deforms the cell thereby causing a perturbation of the cell such that a tolerogenic factor enters the immune cell, thereby generating an immunosuppressive immune cell.

Certain aspects of the present invention provide a method of generating a tolerogenic antigen-presenting immune cell, wherein a tolerogenic immune cell is further passed through a second constriction, wherein said second constriction deforms the cell thereby causing a perturbation of the cell such that an antigen enters the immune cell, wherein the antigen is presented by the tolerogenic immune cell. The immune cell is a B cell or a T cell, wherein the antigen is: (i) a polypeptide antigen; (ii) a disease-associated antigen; (iii) a foreign antigen; (iv) a self-antigen; (v) a therapeutic agent; (vi) an allograft transplantation antigen; (vii) a non-protein antigen; (viii) an antigen associated with a virus; or (ix) an adenovirus, adeno-associated virus, baculovirus, herpes virus, or retrovirus, wherein an immune response is suppressed by at least about 10% and wherein the suppressed immune response comprises decreased production and/or secretion of one or more inflammatory cytokines, wherein the tolerogenic immune cell comprises intracellularly a tolerogenic factor comprising a nucleic acid encoding an arginase-1 (ARG1), a nitric-oxide synthase 2 (NOS2), an indoleamine 2,3-dioxygenase (IDO), an IL-4, an IL-10, an IL-13, a transforming growth factor-beta (TGF-β), an IL-35, or an IFN-α, and wherein the tolerogenic immune cell induces an immunological unresponsiveness or decreased responsiveness to challenge with the antigen.

Certain aspects of the present invention provide a method of generating an immunosuppressive antigen-presenting immune cell, wherein an immunosuppressive immune cell is further passed through a second constriction, wherein said second constriction deforms the cell thereby causing a perturbation of the cell such that an antigen enters the immune cell, wherein the antigen is presented by the immunosuppressive immune cell. The immune cell is a B cell or a T cell, wherein the antigen is: (i) a polypeptide antigen; (ii) a disease-associated antigen; (iii) a foreign antigen; (iv) a self-antigen; (v) a therapeutic agent; (vi) an allograft transplantation antigen; (vii) a non-protein antigen; (viii) an antigen associated with a virus; or (ix) an adenovirus, adeno-associated virus, baculovirus, herpes virus, or retrovirus, wherein an immune response is suppressed by at least about 10% and wherein the suppressed immune response comprises decreased production and/or secretion of one or more inflammatory cytokines, wherein the immunosuppressive immune cell comprises intracellularly a tolerogenic factor comprising a nucleic acid encoding an arginase-1 (ARG1), a nitric-oxide synthase 2 (NOS2), an indoleamine 2,3-dioxygenase (IDO), an IL-4, an IL-10, an IL-13, a transforming growth factor-beta (TGF-β), an IL-35, or an IFN-α, and wherein the immunosuppressive immune cell induces an immunological unresponsiveness or decreased responsiveness to challenge with the antigen.

Certain aspects of the present disclosure provide a method of generating a tolerogenic antigen-presenting cell, wherein an antigen-presenting cell is passed through a constriction, wherein said constriction deforms the cell thereby causing a perturbation of the cell such that a tolerogenic factor enters the antigen-presenting cell. In some embodiments, the tolerogenic factor modulates expression and/or activity of an immunomodulatory agent (such as an immunostimulatory agent *(e.g.,* a costimulatory molecule), an immunosuppressive agent, or an inflammatory or anti-inflammatory molecule). In some embodiments, the antigen presenting cell is generated in vitro or in vivo prior to introduction of the immunosuppressive molecule.

Certain aspects of the present disclosure provide a method of generating a immunosuppressive antigen-presenting cell, wherein an antigen-presenting cell is passed through a constriction, wherein said constriction deforms the cell thereby causing a perturbation of the cell such that a tolerogenic factor enters the antigen-presenting cell. In some embodiments, the tolerogenic factor modulates expression and/or activity of an immunomodulatory agent (such as an immunostimulatory agent *(e.g.,* a costimulatory molecule), an immunosuppressive agent, or an inflammatory or anti-inflammatory molecule). In some embodiments, the antigen presenting cell is generated in vitro or in vivo prior to introduction of the tolerogenic factor.

Certain aspects of the present disclosure provide a method for delivering a tolerogenic factor that generates a tolerogenic phenotype into an immune cell, the method comprising passing a cell suspension comprising the immune cell through a constriction, wherein said constriction deforms the immune cell, thereby causing a perturbation of the cell such that the tolerogenic factor enters the cell, wherein said cell suspension is contacted with the tolerogenic factor. In some embodiments, the tolerogenic factor modulates expression and/or activity of an immunomodulatory agent (such as an immunostimulatory agent (*e.g.,* a costimulatory molecule), an immunosuppressive agent, or an inflammatory or anti-inflammatory molecule)

Certain aspects of the present disclosure provide a method for delivering a tolerogenic factor that generates an immunosuppressive phenotype into an immune cell, the method comprising passing a cell suspension comprising the immune cell through a constriction, wherein said constriction deforms the immune cell, thereby causing a perturbation of the cell such that the tolerogenic factor that generates an immunosuppressive phenotype enters the cell, wherein said cell suspension is contacted with the tolerogenic factor. In some embodiments, the tolerogenic factor modulates expression and/or activity of an immunomodulatory agent (such as an immunostimulatory agent (*e.g.,* a costimulatory molecule), an immunosuppressive agent, or an inflammatory or anti-inflammatory molecule).

Certain aspects of the present invention provide a method for suppressing an immune response in an individual, comprising passing a first cell suspension comprising a first immunosuppressive immune cell through a constriction, wherein said constriction deforms the cell thereby causing a perturbation of the cell such that an antigen enters the immune cell, passing a second cell suspension comprising a second immune cell through a constriction, wherein said constriction deforms the cell thereby causing a perturbation of the cell such that a tolerogenic factor enters the immune cell, wherein an immunosuppressive immune cell is generated, and introducing the first immune cell and second immune cell into the individual, wherein presentation of said antigen by said immunosuppressive immune cell suppresses an immune response to the antigen. The immune cell is a B cell or a T cell, wherein the antigen is: (i) a polypeptide antigen; (ii) a disease-associated antigen; (iii) a foreign antigen; (iv) a self-antigen; (v) a therapeutic agent; (vi) an allograft transplantation antigen; (vii) a non-protein antigen; (viii) an antigen associated with a virus; or (ix) an adenovirus, adeno-associated virus, baculovirus, herpes virus, or retrovirus, wherein an immune response is suppressed by at least about 10% and wherein the suppressed immune response comprises decreased production and/or secretion of one or more inflammatory cytokines, wherein the the immunosuppressive immune cell comprises intracellularly a tolerogenic factor comprising a nucleic acid encoding an arginase-1 (ARG1), a nitric-oxide synthase 2 (NOS2), an indoleamine 2,3-dioxygenase (IDO), an IL-4, an IL-10, an IL-13, a transforming growth factor-beta (TGF-β), an IL-35, or an IFN-α, and wherein the immunosuppressive immune cell induces an immunological unresponsiveness or decreased responsiveness to challenge with the antigen. In some embodiments, the first immune cell and the second immune cell are introduced into the individual simultaneously. In some embodiments, the first immune cell and the second immune cell are introduced into the individual sequentially. In some embodiments, the tolerogenic factor modulates expression and/or activity of an immunomodulatory agent (such as an immunostimulatory agent *(e.g.,* a costimulatory molecule), an immunosuppressive agent, or an inflammatory or anti-inflammatory molecule).

Certain aspects of the present invention provide a method for inducing antigen-specific tolerance in an individual, comprising passing a first cell suspension comprising a first tolerogenic immune cell through a constriction, wherein said constriction deforms the cell thereby causing a perturbation of the cell such that an antigen enters the immune cell, passing a second cell suspension comprising a second immune cell through a constriction, wherein said constriction deforms the cell thereby causing a perturbation of the cell such that a tolerogenic factor enters the immune cell, wherein a tolerogenic immune cell is generated, and introducing the first immune cell and second immune cell into the individual, wherein presentation of said antigen by said tolerogenic immune cell induces tolerance to the antigen. The immune cell is a B cell or a T cell, wherein the antigen is: (i) a polypeptide antigen; (ii) a disease-associated antigen; (iii) a foreign antigen; (iv) a self-antigen; (v) a therapeutic agent; (vi) an allograft transplantation antigen; (vii) a non-protein antigen; (viii) an antigen associated with a virus; or (ix) an adenovirus, adeno-associated virus, baculovirus, herpes virus, or retrovirus, wherein an immune response is suppressed by at least about 10% and wherein the suppressed immune response comprises decreased production and/or secretion of one or more inflammatory cytokines, wherein the tolerogenic immune cell comprises intracellularly a tolerogenic factor comprising a nucleic acid encoding an arginase-1 (ARG1), a nitric-oxide synthase 2 (NOS2), an indoleamine 2,3-dioxygenase (IDO), an IL-4, an IL-10, an IL-13, a transforming growth factor-beta (TGF-β), an IL-35, or an IFN-α, and wherein the tolerogenic immune cell induces an immunological unresponsiveness or decreased responsiveness to challenge with the antigen. In some embodiments, the first immune cell and the second immune cell are introduced into the individual simultaneously. In some embodiments, the first immune cell and the second immune cell are introduced into the individual sequentially. In some embodiments, the tolerogenic factor modulates expression and/or activity of an immunomodulatory agent (such as an immunostimulatory agent (*e.g.,* a costimulatory molecule), an immunosuppressive agent, or an inflammatory or anti-inflammatory molecule).

Certain aspects of the present disclosure provide a method for suppressing an immune response in an individual, the method comprising passing a cell suspension comprising an immune cell through a constriction, wherein said constriction deforms the cell thereby causing a perturbation of the cell such that a compound encoding a nonfunctional cytokine binding protein enters the immune cell, and introducing the immune cell into the individual, wherein said nonfunctional cytokine binding protein is expressed, wherein said nonfunctional cytokine binding protein binds free inflammatory cytokines, thereby suppressing an immune response, for example by inhibiting an inflammatory immune cell. In some embodiments, the nonfunctional cytokine binding protein comprises a nonfunctional cytokine receptor. In some embodiments, the nonfunctional cytokine receptor lacks cytoplasmic signaling domains. In some embodiments, the nonfunctional cytokine binding protein comprises a proteolytic site that cleaves the target cytokine. In some embodiments, the nonfunctional cytokine binding protein comprises an anti-cytokine antibody. In some embodiments, the nonfunctional cytokine binding protein comprises an anti-cytokine B cell receptor.

In some embodiments that can be combined with the previous embodiments, the immune cell is from the individual. In some embodiments, the immune cell is from a different individual.

In some embodiments that can be combined with the previous embodiments, the constriction is contained within a microfluidic channel. In some embodiments, the constriction is a pore or contained within a pore. In some embodiments, the pore is contained in a surface. In some embodiments, the surface is a filter. In some embodiments, the surface is a membrane. In some embodiments, the constriction size is a function of the diameter of the immune cell. In some embodiments, the constriction size is about 20%, about 30%, about 40%, about 50%, about 60%, about 70%, about 80%, about 90%, or about 99% of the cell diameter. In some embodiments, the channel comprises a constriction length of about 10 µm and a constriction width of about 4 µm. In some the pore size is about 0.4µm, about 3µm, about 4µm, about 5µm, about 8µm, about 10µm, about 12µm, or about 14µm. In some embodiments, the method is performed between about -5°C and about 45°C.

Reference to a diameter of an anucleate cell means the diameter of the cell in fluid prior to being passed through a constriction, e.g., as the cell approaches the constriction, unless otherwise specified.

In some embodiments that can be combined with the previous embodiments, the cell suspension comprises a mixed cell population. In some embodiments, the cell suspension is whole blood. In some embodiments, the cell suspension comprises peripheral blood mononuclear cells. In some embodiments, the cell suspension comprises a purified cell population. In some embodiments, the cell suspension comprises mammalian cells. In some embodiments, the cell suspension comprises monkey, mouse, dog, cat, horse, rat, sheep, goat, pig, or rabbit cells. In some embodiments, the cell suspension comprises human cells. In some embodiments, the cell suspension comprises non-mammalian cells. In some embodiments, the cell suspension comprises bacteria, yeast, chicken, frog, insect, fish, or nematode cells. In some embodiments, the immune cell is a mammalian cell. In some embodiments, the immune cell is a monkey, mouse, dog, cat, horse, rat, sheep, goat, pig, or rabbit cell. In some embodiments, the immune cell is a human cell. The immune cell is a T cell or a B cell. In some embodiments, the antigen-presenting cell is a mammalian cell. In some embodiments, the antigen-presenting cell is a monkey, mouse, dog, cat, horse, rat, sheep, goat, pig, or rabbit cell. The antigen-presenting cell is a T cell or a B cell.

In some embodiments that can be combined with the previous embodiments, the antigen is a foreign antigen. In some embodiments, the antigen is a self-antigen. In some embodiments, the antigen is an allograft transplantation antigen. In some embodiments, the antigen is a modified antigen. In some embodiments, the modified antigen comprises an antigen fused with a therapeutic agent. In some embodiments, the modified antigen comprises an antigen fused with a targeting peptide. In some embodiments, said cell suspension is contacted with the antigen before, concurrently, or after passing through the constriction.

In some embodiments that can be combined with the previous embodiments, the tolerogenic factor inhibits the activity of a costimulatory molecule. In some embodiments, the tolerogenic factor decreases expression of a costimulatory molecule. In some embodiments, the tolerogenic factor deletes nucleic acid that modulates expression of the costimulatory molecule. In some embodiments, the tolerogenic factor inhibits the costimulatory molecule. In some embodiments, the tolerogenic factor increases the activity of a transcriptional regulator that suppresses expression of the costimulatory molecule. In some embodiments, the tolerogenic factor increases the activity of a protein inhibitor that suppresses expression of the costimulatory molecule. In some embodiments, the tolerogenic factor comprises nucleic acid encoding a suppressor of the costimulatory molecule. In some embodiments, the costimulatory molecule is CD80 or CD86.

In some embodiments that can be combined with the previous embodiments, the tolerogenic factor enhances the activity of an immunosuppressive factor. In some embodiments, the immunosuppressive factor is a co-inhibitory molecule, a transcriptional regulator, or an immunosuppressive molecule. In some embodiments, the tolerogenic factor enhances the activity of the co-inhibitory molecule. In some embodiments, the tolerogenic factor increases expression of a co-inhibitory molecule. In some embodiments, the tolerogenic factor encodes the co-inhibitory molecule. In some embodiments, the tolerogenic factor increases the activity of the co-inhibitory molecule. In some embodiments, the tolerogenic factor increases the activity of a transcriptional regulator that enhances expression of the co-inhibitory molecule. In some embodiments, the tolerogenic factor increases the activity of a polypeptide that increases expression of the co-inhibitory molecule. In some embodiments, the tolerogenic factor comprises nucleic acid encoding an enhancer of the co-inhibitory molecule. In some embodiments, the co-inhibitory molecule is PD-L1, PD-L2, or CTLA-4.

In some embodiments, the tolerogenic factor enhances the activity of the immunosuppressive molecule. In some embodiments, the tolerogenic factor increases expression of an immunosuppressive molecule. In some embodiments, the tolerogenic factor encodes the immunosuppressive molecule. In some embodiments, the tolerogenic factor increases the activity of the immunosuppressive molecule. In some embodiments, the tolerogenic factor increases the activity of a transcriptional regulator that enhances expression of the immunosuppressive molecule. In some embodiments, the tolerogenic factor increases the activity of a polypeptide that enhances expression of the immunosuppressive molecule. The tolerogenic factor comprises nucleic acid encoding an arginase-1 (ARG1), a nitric-oxide synthase 2 (NOS2), an indoleamine 2,3-dioxygenase (IDO), an IL-4, an IL-10, an IL-13, an IL-35, an IFNα, or a TGFβ.

In some embodiments, the tolerogenic factor inhibits the activity of an inflammatory molecule. In some embodiments, the inflammatory molecule is an inflammatory transcription factor. In some embodiments, the tolerogenic factor inhibits the inflammatory transcription factor. In some embodiments, the tolerogenic factor decreases expression of an inflammatory transcription factor. In some embodiments, the tolerogenic factor deletes nucleic acid encoding the inflammatory transcription factor. In some embodiments, the tolerogenic factor increases the activity of a transcriptional regulator that suppresses expression of the inflammatory transcription factor. In some embodiments, the tolerogenic factor increases the activity of a protein inhibitor that suppresses expression of the inflammatory transcription factor. In some embodiments, the tolerogenic factor comprises nucleic acid encoding a suppressor of the inflammatory transcription factor. In some embodiments, the inflammatory transcription factor is NF-kB, an interferon regulatory factor, or a molecule associated with the JAK-STAT signaling pathway. In some embodiments, the tolerogenic factor encodes a modified TCR containing cytoplasmic signaling domain that triggers production of immunosuppressive cytokines upon binding to antigen. In some embodiments, the tolerogenic factor encodes a chimeric antigen receptor containing cytoplasmic signaling domains that trigger production of immunosuppressive cytokines upon binding to antigen.

In some embodiments, the tolerogenic factor comprises a nucleic acid encoding siRNA, mRNA, miRNA, lncRNA, tRNA, or shRNA. In some embodiments, the tolerogenic factor is a plasmid. In some embodiments, the tolerogenic factor comprises a protein-nucleic acid complex. In some embodiments, the tolerogenic factor comprises a Cas polypeptide and a guide RNA or donor DNA. In some embodiments, the tolerogenic factor comprises nucleic acid encoding for a Cas polypeptide and a guide RNA or donor DNA. In some embodiments, the tolerogenic factor comprises a Cas9 polypeptide and a guide RNA or donor DNA. In some embodiments, the tolerogenic factor comprises nucleic acid encoding for a Cas9 polypeptide and a guide RNA or donor DNA. In some embodiments, the tolerogenic factor comprises a polypeptide. In some embodiments, the polypeptide is a nuclease, TALEN protein, Zinc finger nuclease, mega nuclease, or CRE recombinase. In some embodiments, the polypeptide is a transposase or integrase enzyme. In some embodiments, the polypeptide is an antibody. In some embodiments, the polypeptide is a transcription factor. In some embodiments, the tolerogenic factor is a small molecule. In some embodiments, the tolerogenic factor is a nanoparticle. In some embodiments, said cell suspension is contacted with the tolerogenic factor before, concurrently, or after passing through the constriction.

The immune response is suppressed by at least about 10%. In some embodiments, the immune response is suppressed by at least about 25%, about 50%, about 75%, about 100%, about 150%, about 200%, or more than about 200%. The suppressed immune response comprises decreased production and/or secretion of one or more inflammatory cytokines. In some embodiments, the suppressed immune response comprises a decreased T cell response. In some embodiments, the decreased T cell response comprises decreased T cell activation. In some embodiments, the decreased T cell response comprises decreased T cell survival. In some embodiments, the decreased T cell response comprises decreased T cell proliferation. In some embodiments, the decreased T cell response comprises decreased T cell functionality. In some embodiments, the decreased T cell response comprises a change in T cell phenotype. In some embodiments, the suppressed immune response comprises uncostimulated activation of a T cell. In some embodiments, the suppressed immune response comprises an enhanced Treg response. In some embodiments, the suppressed immune response comprises a decreased B cell response. In some embodiments, the decreased B cell response comprises decreased antibody production. In some embodiments, the suppressed immune response comprises decreased cytokine production. In some embodiments, the suppressed immune response comprises a decreased autoimmune response. In some embodiments, the suppressed immune response comprises a decreased allergic response. In some embodiments, the antigen is an antigen associated with transplanted tissue. In some embodiments, the suppressed immune response comprises a decreased immune response against the transplanted tissue. In some embodiments, the antigen is associated with a virus. In some embodiments, the suppressed immune response comprises a decreased pathogenic immune response to the virus. In some embodiments, the antigen is associated with a bacterium. In some embodiments, the suppressed immune response comprises a decreased pathogenic immune response to the bacterium. In some embodiments, the antigen is associated with a fungus. In some embodiments, the suppressed immune response comprises a decreased pathogenic immune response to the fungus. In some embodiments, the suppressed immune response comprises a decreased immune response against a therapeutic agent. In some embodiments, the suppressed immune response comprises a decreased immune response against a therapeutic vehicle.

In embodiments where tolerance is induced, the tolerance may comprise a decreased T cell response. In some embodiments, the decreased T cell response comprises decreased T cell activation. In some embodiments, the decreased T cell response comprises decreased T cell survival. In some embodiments, the decreased T cell response comprises decreased T cell proliferation. In some embodiments, the decreased T cell response comprises decreased T cell functionality. In some embodiments, the decreased T cell response comprises a change in T cell phenotype. In some embodiments, the tolerance comprises uncostimulated activation of a T cell. In some embodiments, the tolerance comprises an enhanced Treg response. In some embodiments, the tolerance comprises a decreased B cell response. In some embodiments, the decreased B cell response comprises decreased antibody production. In some embodiments, the tolerance comprises decreased cytokine production. In some embodiments, the tolerance comprises a decreased autoimmune response. In some embodiments, the tolerance comprises a decreased allergic response. In some embodiments, the tolerance comprises a decreased immune response against the transplanted tissue. In some embodiments, the tolerance comprises a decreased pathogenic immune response to the virus. In some embodiments, the tolerance comprises a decreased immune response against a therapeutic agent. In some embodiments, the tolerance comprises a decreased immune response against a therapeutic vehicle.

Certain aspects of the present disclosure relate to a system comprising the constriction, immune cell, antigen, and/or tolerogenic factor for use in any one of the aforementioned methods. Certain aspects of the present disclosure relate to a system comprising the constriction, immune cell, and compound encoding a nonfunctional cytokine binding protein for use in any one of the aforementioned methods.

### BRIEF DESCRIPTION OF THE DRAWINGS

FIG. 1 shows a schematic of the treatment schedule.
FIG. 2 shows the number of OT-I- and OT-II-specific T cells on day 12 in mice from the four treatment groups.
FIG. 3 shows the percent of splenic T cells that expressed high levels of IFN-γ (left) and the level of IFN-γ production (right).
FIG. 4 shows the number of OT-I T cells producing IFN-γ as determined by ELISpot.
FIG. 5 shows representative flow cytograms for TCRVa2+ splenic OT-I and lymph node OT-II T cells for each treatment group.
FIG. 6 shows representative flow cytograms for OT-I cells with high IFN-γ and CD44 antigen-activation marker.
FIG. 7 shows a schematic of the treatment schedule.
FIG. 8 shows the number of OT-I- and OT-II-specific T cells on day 12 in mice from the four treatment groups.
FIG. 9 shows the percent of splenic T cells that expressed high levels of IFN-γ.
FIG. 10 shows representative flow cytograms for OT-I T cell numbers vs. CD8+ T cells (top panels) and for cells with high IFN-γ vs. CD44 antigen-activation marker (bottom panels).
FIGS. 11A and 11B show schematics of representative treatment schedules.
FIGS. 12A and 12B show schematics of representative treatment schedules.

### DETAILED DESCRIPTION

The invention provides methods for inducing tolerance and/or suppressing an immune response in an individual by passing a cell suspension containing a tolerogenic immune cell or an immunosuppressive immune cell through a constriction, enabling delivery of an antigen to the immune cell. In some embodiments the constriction is contained within a microfluidic channel. In some embodiments the constriction is a pore or contained within a pore.

Certain aspects of the present disclosure relate to methods for inducing tolerance and/or suppressing an immune response to an antigen in an individual, the method comprising passing a cell suspension comprising an immune cell through a constriction, wherein the constriction deforms the cell thereby causing a perturbation of the cell such that an antigen enters the immune cell, and introducing the immune cell into the individual, wherein presentation of the antigen in a tolerogenic environment induces tolerance and/or suppresses an immune response to the antigen. In some embodiments, the antigen is processed in a tolerogenic environment. In some embodiments, the tolerance and/or immune suppression are antigen-specific. In some embodiments, the tolerance and/or immune suppression are non-specific, including tolerance and/or suppression of an immune response to a plurality of antigens.

Certain aspects of the present disclosure relate to methods for inducing tolerance and/or suppressing an immune response to an antigen in an individual, the method comprising passing a cell suspension comprising an immune cell through a constriction, wherein the constriction deforms the cell thereby causing a perturbation of the cell such that an antigen and a tolerogenic factor enter the immune cell, and introducing the immune cell into the individual, thereby inducing tolerance and/or suppressing an immune response to the antigen. In some embodiments, said antigen is presented in a tolerogenic environment. In some embodiments, the tolerogenic factor generates a tolerogenic environment, wherein presentation of the antigen in said tolerogenic environment induces tolerance and/or suppresses an immune response to the antigen. In some embodiments, said antigen is processed in a tolerogenic environment. In some embodiments, the tolerance and/or immune suppression are antigen-specific. In some embodiments, the tolerance and/or immune suppression are non-specific, including tolerance and/or suppression of an immune response to a plurality of antigens.

Certain aspects of the present disclosure relate to methods for inducing tolerance and/or suppressing an immune response to an antigen in an individual, the method comprising passing a cell suspension comprising an immune cell through a constriction, wherein the constriction deforms the cell thereby causing a perturbation of the cell such that a tolerogenic factor enters the immune cell, and introducing the immune cell into the individual, thereby inducing tolerance and/or suppressing an immune response to an antigen. In some embodiments, the tolerogenic factor generates a tolerogenic environment, wherein presentation of an antigen in said tolerogenic environment induces tolerance and/or suppresses an immune response to the antigen. In some embodiments, the tolerance and/or immune suppression are antigen-specific. In some embodiments, the tolerance and/or immune suppression are non-specific, including tolerance and/or suppression of an immune response to a plurality of antigens.

Certain aspects of the present disclosure relate to methods for generating a tolerogenic and/or immunosuppressive antigen-presenting cell, wherein the antigen-presenting cell is passed through a constriction, wherein the constriction deforms the cell thereby causing a perturbation of the cell such that a tolerogenic factor enters the antigen-presenting cell. In some embodiments, the tolerogenic factor modulates expression and/or activity of an immunomodulatory agent (such as an immunostimulatory agent *(e.g.,* a costimulatory molecule), an immunosuppressive agent, or an inflammatory or anti-inflammatory molecule).

### I. GENERAL TECHNIQUES

The techniques and procedures described or referenced herein are generally well understood and commonly employed using conventional methodology by those skilled in the art, such as, for example, the widely utilized methodologies described in Molecular Cloning: A Laboratory Manual (Sambrook et al., 4th ed., Cold Spring Harbor Laboratory Press, Cold Spring Harbor, N.Y., 2012); Current Protocols in Molecular Biology (F.M. Ausubel, et al. eds., 2003); the series Methods in Enzymology (Academic Press, Inc.); PCR 2: A Practical Approach (M.J. MacPherson, B.D. Hames and G.R. Taylor eds., 1995); Antibodies, A Laboratory Manual (Harlow and Lane, eds., 1988); Culture of Animal Cells: A Manual of Basic Technique and Specialized Applications (R.I. Freshney, 6th ed., J. Wiley and Sons, 2010); Oligonucleotide Synthesis (M.J. Gait, ed., 1984); Methods in Molecular Biology, Humana Press; Cell Biology: A Laboratory Notebook (J.E. Cellis, ed., Academic Press, 1998); Introduction to Cell and Tissue Culture (J.P. Mather and P.E. Roberts, Plenum Press, 1998); Cell and Tissue Culture: Laboratory Procedures (A. Doyle, J.B. Griffiths, and D.G. Newell, eds., J. Wiley and Sons, 1993-8*);* Handbook of Experimental Immunology (D.M. Weir and C.C. Blackwell, eds., 1996); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Ausubel et al., eds., J. Wiley and Sons, 2002); Immunobiology (C.A. Janeway et al., 2004); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000*);* Using Antibodies: A Laboratory Manual (E. Harlow and D. Lane, Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 2011).

### II. DEFINITIONS

For purposes of interpreting this specification, the following definitions will apply and whenever appropriate, terms used in the singular will also include the plural and vice versa. In the event that any definition set forth below conflicts with any referenced document, the definition set forth shall control.

As used herein, the singular form "a", "an", and "the" includes plural references unless indicated otherwise.

It is understood that aspects and embodiments of the invention described herein include "comprising," "consisting," and "consisting essentially of" aspects and embodiments.

The term "about" as used herein refers to the usual error range for the respective value readily known to the skilled person in this technical field. Reference to "about" a value or parameter herein includes (and describes) embodiments that are directed to that value or parameter per se.

The term "pore" as used herein refers to an opening, including without limitation, a hole, tear, cavity, aperture, break, gap, or perforation within a material. In some examples, (where indicated) the term refers to a pore within a surface of the present disclosure. In other examples, (where indicated) a pore can refer to a pore in a cell membrane.

The term "membrane" as used herein refers to a selective barrier or sheet containing pores. The term includes a pliable sheetlike structure that acts as a boundary or lining. In some examples, the term refers to a surface or filter containing pores. This term is distinct from the term "cell membrane".

The term "filter" as used herein refers to a porous article that allows selective passage through the pores. In some examples the term refers to a surface or membrane containing pores.

The term "heterogeneous" as used herein refers to something which is mixed or not uniform in structure or composition. In some examples the term refers to pores having varied sizes, shapes or distributions within a given surface.

The term "homogeneous" as used herein refers to something which is consistent or uniform in structure or composition throughout. In some examples the term refers to pores having consistent sizes, shapes, or distribution within a given surface.

The term "heterologous" as used herein refers to a molecule which is derived from a different organism. In some examples the term refers to a nucleic acid or protein which is not normally found or expressed within the given organism.

As used herein, the term "inhibit" may refer to the act of blocking, reducing, eliminating, or otherwise antagonizing the presence, or an activity of, a particular target. Inhibition may refer to partial inhibition or complete inhibition. For example, inhibiting an immune response may refer to any act leading to a blockade, reduction, elimination, or any other antagonism of an immune response. In other examples, inhibition of the expression of a nucleic acid may include, but not limited to reduction in the transcription of a nucleic acid, reduction of mRNA abundance (*e.g*., silencing mRNA transcription), degradation of mRNA, inhibition of mRNA translation, and so forth. In other examples, inhibition of the expression of a protein may include, but not be limited to, reduction in the transcription of a nucleic acid encoding the protein, reduction in the stability of mRNA encoding the protein, inhibition of translation of the protein, reduction in stability of the protein, and so forth.

As used herein, the term "suppress" may refer to the act of decreasing, reducing, prohibiting, limiting, lessening, or otherwise diminishing the presence, or an activity of, a particular target. Suppression may refer to partial suppression or complete suppression. For example, suppressing an immune response may refer to any act leading to decreasing, reducing, prohibiting, limiting, lessening, or otherwise diminishing an immune response. In other examples, suppression of the expression of a nucleic acid may include, but not limited to reduction in the transcription of a nucleic acid, reduction of mRNA abundance (*e.g*., silencing mRNA transcription), degradation of mRNA, inhibition of mRNA translation, and so forth. In other examples, suppression of the expression of a protein may include, but not be limited to, reduction in the transcription of a nucleic acid encoding the protein, reduction in the stability of mRNA encoding the protein, inhibition of translation of the protein, reduction in stability of the protein, and so forth.

As used herein, the term "enhance" may refer to the act of improving, boosting, heightening, or otherwise increasing the presence, or an activity of, a particular target. For example, enhancing an immune response may refer to any act leading to improving, boosting, heightening, or otherwise increasing an immune response. In other examples, enhancing the expression of a nucleic acid may include, but not limited to increase in the transcription of a nucleic acid, increase in mRNA abundance (*e.g.*, increasing mRNA transcription), decrease in degradation of mRNA, increase in mRNA translation, and so forth. In other examples, enhancing the expression of a protein may include, but not be limited to, increase in the transcription of a nucleic acid encoding the protein, increase in the stability of mRNA encoding the protein, increase in translation of the protein, increase in the stability of the protein, and so forth.

As used herein, the term "induce" may refer to the act of initiating, prompting, stimulating, establishing, or otherwise producing a result. For example, inducing an immune response may refer to any act leading to initiating, prompting, stimulating, establishing, or otherwise producing a desired immune response. In other examples, inducing the expression of a nucleic acid may include, but not limited to initiation of the transcription of a nucleic acid, initiation of mRNA translation, and so forth. In other examples, inducing the expression of a protein may include, but not be limited to, increase in the transcription of a nucleic acid encoding the protein, increase in the stability of mRNA encoding the protein, increase in translation of the protein, increase in the stability of the protein, and so forth.

The term "homologous" as used herein refers to a molecule which is derived from the same organism. In some examples the term refers to a nucleic acid or protein which is normally found or expressed within the given organism.

The term "polynucleotide" or "nucleic acid" as used herein refers to a polymeric form of nucleotides of any length, including ribonucleotides and deoxyribonucleotides. Thus, this term includes, but is not limited to, single-, double- or multi-stranded DNA or RNA, genomic DNA, cDNA, DNA-RNA hybrids, or a polymer comprising purine and pyrimidine bases, or other natural, chemically or biochemically modified, non-natural, or derivatized nucleotide bases. The backbone of the polynucleotide can comprise sugars and phosphate groups (as may typically be found in RNA or DNA), or modified or substituted sugar or phosphate groups. The backbone of the polynucleotide can comprise repeating units, such as N-(2-aminoethyl)-glycine, linked by peptide bonds (*i.e.,* peptide nucleic acid). Alternatively, the backbone of the polynucleotide can comprise a polymer of synthetic subunits such as phosphoramidates and thus can be an oligodeoxynucleoside phosphoramidate (P-NH2) or a mixed phosphoramidate-phosphodiester oligomer. In addition, a double-stranded polynucleotide can be obtained from the single stranded polynucleotide product of chemical synthesis either by synthesizing the complementary strand and annealing the strands under appropriate conditions, or by synthesizing the complementary strand de novo using a DNA polymerase with an appropriate primer.

The terms "polypeptide" and "protein" are used interchangeably to refer to a polymer of amino acid residues, and are not limited to a minimum length. Such polymers of amino acid residues may contain natural or non-natural amino acid residues, and include, but are not limited to, peptides, oligopeptides, dimers, trimers, and multimers of amino acid residues. Both full-length proteins and fragments thereof are encompassed by the definition. The terms also include post-expression modifications of the polypeptide, for example, glycosylation, sialylation, acetylation, phosphorylation, and the like. Furthermore, for purposes of the present invention, a "polypeptide" refers to a protein which includes modifications, such as deletions, additions, and substitutions (generally conservative in nature), to the native sequence, as long as the protein maintains the desired activity. These modifications may be deliberate, as through site-directed mutagenesis, or may be accidental, such as through mutations of hosts which produce the proteins or errors due to PCR amplification.

For any of the structural and functional characteristics described herein, methods of determining these characteristics are known in the art.

### III. IMMUNE SUPPRESSION AND TOLERANCE

In certain aspects, the invention provides methods for suppressing an immune response and/or inducing tolerance in an individual, the method comprising passing a cell suspension comprising an immune cell through a constriction, wherein said constriction deforms the cell thereby causing a perturbation of the cell such that an antigen and/or a tolerogenic factor enter the immune cell, wherein a tolerogenic or immunosuppressive immune cell is generated, and introducing the immune cell into the individual, wherein presentation of said antigen induces tolerance and/or suppresses an immune response to the antigen.

A tolerogenic immune cell is characterized as an immune cell capable or presenting an antigen in a tolerogenic manner (*e.g*., presentation of the antigen by the tolerogenic immune cell induces tolerance to the antigen), or capable of conferring a tolerogenic phenotype on another cell, such as another antigen-presenting cell, thereby generating a tolerogenic antigen-presenting cell. An immunosuppressive immune cell is characterized as an immune cell capable of presenting an antigen in an immunosuppressive manner (*e.g*., presentation of the antigen by the immunosuppressive immune cell suppresses an immune response to the antigen), or capable of conferring an immunosuppressive phenotype on another cell, such as another antigen-presenting cell, thereby generating an immunosuppressive antigen-presenting cell.

In some embodiments, a tolerogenic and/or immunosuppressive immune cell described herein, or a precursor thereof, was not contacted with an adjuvant. In some embodiments, the adjuvant includes ligands for pattern recognition receptors (PRR) (*e.g.,* toll-like receptors (TLRs), NOD-like receptors (NLRs), RIG-I-like receptors (RLRs), and C-type lectin receptors (CLRs)), and pathogen-associated molecular patterns (PAMPs). In some embodiments, the adjuvant is selected from the group consisting of TLR3 and RLR ligands (*e.g.,* synthetic analogs of dsRNA, such as poly(I:C)), TLR4 ligands (*e.g*., lipopolysaccharides (LPS), monophosphoryl lipid A (MPLA)), TLR5 ligands (*e.g*., bacterial flagellin (alone or fused to antigen)), TLR7/8 ligands (*e.g*., imidazoquinolines such as imiquimod, gardiquimod, and R848), TLR9 ligands (*e.g*., oligodeoxynucleotides containing specific CpG motifs, including CpG ODNs such as ODN 1826 and ODN 2006), NOD2 ligands (*e.g*., bacterial cell wall fragments such as muramyl dipeptide (MDP)), alum, water-in-oil emulsions (Freund's Incomplete Adjuvant, MF59), rhIL-2, anti-CD40 or CD40L, IL-12, and cyclic dinucleotides.

In some embodiments, a tolerogenic and/or immunosuppressive immune cell described herein comprises a reduced ability to provide one or more costimulatory signals as compared to a non-tolerogenic and/or non-immunosuppressive precursor. In some embodiments, the costimulatory signals including signaling through CD40, CD80, CD86, CD54, CD83, CD79, or ICOS ligand.

In some embodiments, a tolerogenic and/or immunosuppressive immune cell described herein comprises a reduced ability to provide one or more inflammatory signals as compared to a non-tolerogenic and/or non-immunosuppressive precursor. In some embodiments, the inflammatory signals including signaling through interleukin-1 (IL-1), IL-12, IL-18, tumor necrosis factor (TNF), interferon gamma (IFN-gamma), granulocyte-macrophage colony stimulating factor (GM-CSF), NF-κB, an interferon regulatory factor (IRF), or JAK-STAT.

Immunological tolerance is an immunological unresponsiveness to challenge with the antigen to which tolerance has been induced. Tolerance is demonstrated when a subject is unresponsive to subsequent to challenge with the tolerance-inducing antigen. Tolerance can be mediated by several different mechanisms which include apoptosis of the autoreactive cells, induction of anergy, or deviation of lymphocyte phenotype. In some embodiments, the tolerance may include T cell non-responsiveness, causing deactivation of immune response to a specific antigen. Tolerance can also be mediated by regulatory T cells (Tregs) through secretion of immunosuppressive cytokines and via contact-dependent interactions with cell surface molecules or cytotoxic factors. Defects in tolerogenic mechanisms can contribute to autoimmune diseases, transplantation rejection, anti-drug responses, and pathogenic responses to viral infection. Immunosuppression is a decrease in the activity of the immune response. For example, immunosuppression may include, without limitation, decreased responsiveness to challenge with antigen, decreased immune cell activation and proliferation, modulated cytokine production and/or secretion, decreased immune cell survival, or decreased immune cell effector functions.

In some embodiments, the invention provides methods for delivery to an immune cell, wherein the cell is a mammalian cell. In some embodiments, the cell is a primary cell or a cell line cell. In some embodiments, the cell is a blood cell. The immune cell is a T cell or a B cell In some embodiments, the immune cell is an ex vivo generated cell (e.g. an ex vivo generated dendritic cell). Exemplary ex vivo generated cells include immune cells generated from stem cells or precursor cells (e.g., autologous stem cells or precursor cells). In some embodiments, the immune cell is a memory cell. In some embodiments, the immune cell is a primary human T cell. In some embodiments, the cell is a mouse, dog, cat, horse, rat, goat, monkey, or rabbit cell. In some embodiments, the cell is a human cell. In some embodiments, the cell suspension comprises non-mammalian cell. In some embodiments, the cell is a chicken, frog, insect, fish, or nematode cell.

In certain aspects, the invention provides methods for inducing tolerance to an antigen in an individual, the method comprising passing a cell suspension comprising an immune cell through a constriction, wherein said constriction deforms the cell thereby causing a perturbation of the cell such that an antigen contacted with the cell enters the immune cell, and introducing the immune cell into the individual, wherein the immune cell is a tolerogenic immune cell, and wherein presentation of said antigen induces tolerance to the antigen. In some embodiments, the antigen is presented by the tolerogenic immune cell to induce tolerance to the antigen. In some embodiments, the tolerogenic immune cell confers a tolerogenic phenotype on another antigen-presenting cell, thereby generating a tolerogenic antigen-presenting cell, and the antigen is presented by the tolerogenic antigen-presenting cell to induce tolerance to the antigen. In some embodiments, the tolerance is antigen-specific.

In certain aspects, the invention provides methods for inducing tolerance to an antigen in an individual, the method comprising passing a cell suspension comprising an immune cell through a constriction, wherein said constriction deforms the cell thereby causing a perturbation of the cell such that an antigen and a tolerogenic factor contacted with the cell enter the immune cell, thereby generating a tolerogenic immune cell comprising the antigen, and introducing the tolerogenic immune cell into the individual, wherein presentation of said antigen induces tolerance to the antigen. In some embodiments, the antigen is presented by the tolerogenic immune cell to induce tolerance to the antigen. In some embodiments, the tolerogenic immune cell confers a tolerogenic phenotype on another antigen-presenting cell, thereby generating a tolerogenic antigen-presenting cell, and the antigen is presented by the tolerogenic antigen-presenting cell to induce tolerance to the antigen. In some embodiments, the tolerance is antigen-specific.

In certain aspects, the invention provides methods for suppressing an immune response to an antigen in an individual, the method comprising passing a cell suspension comprising an immune cell through a constriction, wherein said constriction deforms the cell thereby causing a perturbation of the cell such that an antigen contacted with the cell enters the immune cell, and introducing the immunosuppressive immune cell into the individual, wherein the immune cell is an immunosuppressive immune cell, and wherein presentation of said antigen suppresses an immune response to the antigen. In some embodiments, the antigen is presented by the immunosuppressive immune cell to suppress an immune response to the antigen. In some embodiments, the immunosuppressive immune cell confers an immunosuppressive phenotype on another antigen-presenting cell, thereby generating an immunosuppressive antigen-presenting cell, and the antigen is presented by the immunosuppressive antigen-presenting cell to suppress an immune response to the antigen. In some embodiments, the immunosuppression is antigen-specific.

In certain aspects, the invention provides methods for suppressing an immune response to an antigen in an individual, the method comprising passing a cell suspension comprising an immune cell through a constriction, wherein said constriction deforms the cell thereby causing a perturbation of the cell such that an antigen and a tolerogenic factor contacted with the cell enter the immune cell, thereby generating an immunosuppressive immune cell comprising the antigen, and introducing the immunosuppressive immune cell into the individual, wherein presentation of said antigen suppresses an immune response to the antigen. In some embodiments, the antigen is presented by the immunosuppressive immune cell to suppress an immune response to the antigen. In some embodiments, the immunosuppressive immune cell confers an immunosuppressive phenotype on another antigen-presenting cell, thereby generating an immunosuppressive antigen-presenting cell, and the antigen is presented by the immunosuppressive antigen-presenting cell to suppress an immune response to the antigen. In some embodiments, the immunosuppression is antigen-specific.

In certain aspects, the invention provides methods for inducing tolerance to an antigen in an individual, the method comprising introducing a tolerogenic immune cell into the individual, wherein the tolerogenic immune cell comprises an antigen, wherein presentation of said antigen induces tolerance to the antigen, and wherein the antigen was introduced to the tolerogenic immune cell by passing the tolerogenic immune cell or a precursor thereof through a constriction, wherein said constriction deformed the cell thereby causing a perturbation of the cell such that the antigen entered the tolerogenic immune cell or precursor thereof. In some embodiments, the antigen is presented by the tolerogenic immune cell to induce tolerance to the antigen. In some embodiments, the tolerogenic immune cell confers a tolerogenic phenotype on another antigen-presenting cell, thereby generating a tolerogenic antigen-presenting cell, and the antigen is presented by the tolerogenic antigen-presenting cell to induce tolerance to the antigen. In some embodiments, the tolerance is antigen-specific.

In certain aspects, the invention provides methods for inducing tolerance to an antigen in an individual, the method comprising introducing a tolerogenic immune cell into the individual, wherein the tolerogenic immune cell comprises an antigen and a tolerogenic factor, wherein presentation of said antigen induces tolerance to the antigen, and wherein the antigen and the tolerogenic factor were introduced to the tolerogenic immune cell by passing the tolerogenic immune cell or a precursor thereof through a constriction, wherein said constriction deformed the cell thereby causing a perturbation of the cell such that the antigen and the tolerogenic factor entered the tolerogenic immune cell or precursor thereof. In some embodiments, the antigen is presented by the tolerogenic immune cell to induce tolerance to the antigen. In some embodiments, the tolerogenic immune cell confers a tolerogenic phenotype on another antigen-presenting cell, thereby generating a tolerogenic antigen-presenting cell, and the antigen is presented by the tolerogenic antigen-presenting cell to induce tolerance to the antigen. In some embodiments, the tolerance is antigen-specific.

In certain aspects, the invention provides methods for suppressing an immune response to an antigen in an individual, the method comprising introducing an immunosuppressive immune cell into the individual, wherein the immunosuppressive immune cell comprises an antigen, wherein presentation of said antigen suppresses an immune response to the antigen, and wherein the antigen was introduced to the immunosuppressive immune cell by passing the immunosuppressive immune cell or a precursor thereof through a constriction, wherein said constriction deformed the cell thereby causing a perturbation of the cell such that the antigen entered the immunosuppressive immune cell or precursor thereof. In some embodiments, the antigen is presented by the immunosuppressive immune cell to suppress an immune response to the antigen. In some embodiments, the immunosuppressive immune cell confers an immunosuppressive phenotype on another antigen-presenting cell, thereby generating an immunosuppressive antigen-presenting cell, and the antigen is presented by the immunosuppressive antigen-presenting cell to suppress an immune response to the antigen. In some embodiments, the immunosuppression is antigen-specific.

In certain aspects, the invention provides methods for suppressing an immune response to an antigen in an individual, the method comprising introducing an immunosuppressive immune cell into the individual, wherein the immunosuppressive immune cell comprises an antigen and a tolerogenic factor, wherein presentation of said antigen suppresses an immune response to the antigen, and wherein the antigen and the tolerogenic factor were introduced to the immunosuppressive immune cell by passing the immunosuppressive immune cell or a precursor thereof through a constriction, wherein said constriction deformed the cell thereby causing a perturbation of the cell such that the antigen and the tolerogenic factor entered the immunosuppressive immune cell or precursor thereof. In some embodiments, the antigen is presented by the immunosuppressive immune cell to suppress an immune response to the antigen. In some embodiments, the immunosuppressive immune cell confers an immunosuppressive phenotype on another antigen-presenting cell, thereby generating an immunosuppressive antigen-presenting cell, and the antigen is presented by the immunosuppressive antigen-presenting cell to suppress an immune response to the antigen. In some embodiments, the immunosuppression is antigen-specific.

In certain aspects, the invention provides methods for generating a tolerogenic immune cell, the method comprising passing a cell suspension comprising an immune cell through a constriction, wherein said constriction deforms the cell thereby causing a perturbation of the cell such that a tolerogenic factor contacted with the cell enters the immune cell, thereby generating the tolerogenic immune cell. In certain aspects, the invention provides methods for generating a tolerogenic antigen-presenting immune cell, wherein the tolerogenic immune cell is further passed through a second constriction, wherein said second constriction deforms the cell thereby causing a perturbation of the cell such that an antigen contacted with the cell enters the immune cell, wherein the antigen is presented by the tolerogenic immune cell.

In certain aspects, the invention provides methods for generating an immunosuppressive immune cell, the method comprising passing a cell suspension comprising an immune cell through a constriction, wherein said constriction deforms the cell thereby causing a perturbation of the cell such that a tolerogenic factor contacted with the cell enters the immune cell, thereby generating the immunosuppressive immune cell. In certain aspects, the invention provides methods for generating an immunosuppressive antigen-presenting immune cell, wherein the immunosuppressive immune cell is further passed through a second constriction, wherein said second constriction deforms the cell thereby causing a perturbation of the cell such that an antigen contacted with the cell enters the immune cell, wherein the antigen is presented by the immunosuppressive immune cell.

In some embodiments, the immune suppression and/or tolerance comprises modulation of the expression and/or activity of an immunomodulatory agent *(e.g.,* a costimulatory molecule, an immunosuppressive agent, or an inflammatory or anti-inflammatory molecule). In some embodiments, the immune suppression and/or tolerance comprises inhibition of the expression and/or activity of an immunostimulatory agent *(e.g.,* a costimulatory molecule), enhancement of the expression and/or activity of an immunosuppressive agent, inhibition of the expression and/or activity of an inflammatory molecule, and/or enhancement of the expression and/or activity of an anti-inflammatory molecule.

In some embodiments, the immune suppression and/or tolerance comprises inhibition of the expression and/or activity of an immunostimulatory agent *(e.g.,* a costimulatory molecule). In some embodiments, the immunostimulatory agent is a costimulatory molecule. Interaction between costimulatory molecules and their ligands is important to sustain and integrate TCR signaling to stimulate optimal T cell proliferation and differentiation. In some embodiments, the immune suppression and/or tolerance comprises decreased expression of a costimulatory molecule. Exemplary costimulatory molecules expressed on antigen-presenting cells include, without limitation, CD40, CD80, CD86, CD54, CD83, CD79, or ICOS Ligand. In some embodiments, the costimulatory molecule is CD80 or CD86. In some embodiments, the immune suppression and/or tolerance comprises inhibition of the expression of a nucleic acid that expresses or modulates expression of the costimulatory molecule. In some embodiments, the nucleic acid that expresses or modulates expression of the costimulatory molecule is deleted. In some embodiments, deletion of the nucleic acid that expresses or modulates expression of the costimulatory molecule is achieved via gene editing. In some embodiments, the immune suppression and/or tolerance comprises inhibition of the costimulatory molecule. In some embodiments, the immune suppression and/or tolerance comprises inhibition of the expression or function of the costimulatory molecule. In some embodiments, the immune suppression and/or tolerance comprises increased activity of a transcriptional regulator that suppresses expression of the costimulatory molecule. In some embodiments, the immune suppression and/or tolerance comprises increased activity of a protein inhibitor that suppresses expression of the costimulatory molecule. In some embodiments, the immune suppression and/or tolerance comprises degradation of the costimulatory molecule. In some embodiments, the immune suppression and/or tolerance comprises labeling of the costimulatory molecule for destruction. For example, the immune suppression and/or tolerance may comprise ubiquitination of the costimulatory molecule, thereby targeting it for destruction.

In some embodiments, the immune suppression and/or tolerance comprises enhancement of the expression and/or activity of an immunosuppressive agent. In some embodiments, the immunosuppressive agent is a co-inhibitory molecule, a transcriptional regulator, or an immunosuppressive molecule. Co-inhibitory molecules negatively regulate the activation of lymphocytes. Exemplary co-inhibitory molecules include, without limitation, PD-L1, PD-L2, HVEM, B7-H3, TRAIL, immunoglobulin-like transcripts (ILT) receptors (ILT2, ILT3, ILT4), FasL, CTLA4, CD39, CD73, and B7-H4. In some embodiments, the co-inhibitory molecule is PD-L1 or PD-L2. In some embodiments, the immune suppression and/or tolerance comprises increased activity of the co-inhibitory molecule. In some embodiments, the immune suppression and/or tolerance comprises increased expression of a co-inhibitory molecule. In some embodiments, the immune suppression and/or tolerance comprises increased activity of a transcriptional regulator that enhances expression of the co-inhibitory molecule. In some embodiments, the immune suppression and/or tolerance comprises increased activity of a polypeptide that increases expression of the co-inhibitory molecule. In some embodiments, the immune suppression and/or tolerance comprises inhibition of an inhibitor of a co-inhibitory molecule.

In some embodiments, the immune suppression and/or tolerance comprises increased expression and/or activity of an immunosuppressive molecule. Exemplary immunosuppressive molecules include, without limitation, arginase-1 (ARG1), indoleamine 2,3-dioxygenase (IDO), Prostaglandin E2 (PGE2), inducible nitric-oxide synthase (iNOS), nitric oxide (NO), nitric-oxide synthase 2 (NOS2), TSLP, vascular intestinal peptide (VIP), hepatocyte growth factor (HGF), TGFβ, IFNα, IL-4, IL-10, IL-13, and IL-35. In some embodiments, the immunosuppressive molecule is NO or IDO. In some embodiments, the immune suppression and/or tolerance comprises increased activity of the immunosuppressive molecule. In some embodiments, the immune suppression and/or tolerance comprises increased activity of a transcriptional regulator that enhances expression of the immunosuppressive molecule. In some embodiments, the immune suppression and/or tolerance comprises increased activity of a polypeptide that enhances expression of the immunosuppressive molecule. In some embodiments, the immune suppression and/or tolerance comprises inhibition of a negative regulator of an immunosuppressive molecule.

In some embodiments, the immune suppression and/or tolerance comprises inhibition of the expression and/or activity of an inflammatory molecule. In some embodiments, the inflammatory molecule is an inflammatory cytokine. In some embodiments, the inflammatory cytokine is selected from interleukin-1 (IL-1), IL-12, and IL-18, tumor necrosis factor (TNF), interferon gamma (IFN-gamma), and granulocyte-macrophage colony stimulating factor (GM-CSF). In some embodiments, the inflammatory molecule is an inflammatory transcription factor. In some embodiments, the immune suppression and/or tolerance comprises inhibition of the inflammatory transcription factor. In some embodiments, the immune suppression and/or tolerance comprises decreased expression of an inflammatory transcription factor. In some embodiments, the inflammatory transcription factor is NF-κB, an interferon regulatory factor (IRF), or a molecule associated with the JAK-STAT signaling pathway. The NF-κB pathway is a prototypical proinflammatory signaling pathway that mediates the expression of proinflammatory genes including cytokines, chemokines, and adhesion molecules. Interferon regulatory factors (IRFs) constitute a family of transcription factors that can regulate the expression of proinflammatory genes. The JAK-STAT signaling pathway transmits information from extracellular cytokine signals to the nucleus, resulting in DNA transcription and expression of genes involved in immune cell proliferation and differentiation. The JAK-STAT system consists of a cell surface receptor, Janus kinases (JAKs), and Signal Transducer and Activator of Transcription (STAT) proteins. Exemplary JAK-STAT molecules include, without limitation, JAK1, JAK2, JAK 3, Tyk2, STAT1, STAT2, STAT3, STAT4, STATS (STAT5A and STAT5B), and STAT6. In some embodiments, the immune suppression and/or tolerance comprises enhanced expression of a suppressor of cytokine signaling (SOCS) protein. SOCS proteins may inhibit signaling through the JAK-STAT pathway. In some embodiments, the immune suppression and/or tolerance comprises inhibition of the expression of a nucleic acid encoding the inflammatory transcription factor. In some embodiments, the nucleic acid encoding the inflammatory transcription factor is deleted. In some embodiments, the immune suppression and/or tolerance comprises increased activity of a transcriptional regulator that suppresses expression of the inflammatory transcription factor. In some embodiments, the immune suppression and/or tolerance comprises increased activity of a protein inhibitor that suppresses expression of the inflammatory transcription factor.

In some embodiments, the immune suppression and/or tolerance comprises enhancement of the expression and/or activity of an anti-inflammatory molecule. In some embodiments, the anti-inflammatory molecule is an anti-inflammatory cytokine. In some embodiments, the anti-inflammatory cytokine is selected from IL-4, IL-10, IL-13, IFN-alpha and transforming growth factor-beta (TGFβ). In some embodiments, the anti-inflammatory molecule is an anti-inflammatory transcription factor. In some embodiments, the immune suppression and/or tolerance comprises enhancement of the anti-inflammatory transcription factor. In some embodiments, the immune suppression and/or tolerance comprises increased expression of an anti-inflammatory transcription factor. In some embodiments, the immune suppression and/or tolerance comprises enhancement of the expression of a nucleic acid encoding the anti-inflammatory transcription factor. In some embodiments, the nucleic acid encoding the anti-inflammatory transcription factor is deleted. In some embodiments, the immune suppression and/or tolerance comprises decreased activity of a transcriptional regulator that suppresses expression of the anti-inflammatory transcription factor. In some embodiments, the immune suppression and/or tolerance comprises decreased activity of a protein inhibitor that suppresses expression of the anti-inflammatory transcription factor.

In certain aspects, the invention provides methods for generating a tolerogenic antigen-presenting cell comprising an antigen, wherein a tolerogenic antigen-presenting cell is passed through a constriction, wherein said constriction deforms the cell thereby causing a perturbation of the cell such that the antigen enters the tolerogenic antigen-presenting cell, thereby generating the tolerogenic antigen-presenting cell comprising the antigen.

In certain aspects, the invention provides methods for generating a tolerogenic antigen-presenting cell, wherein an antigen-presenting cell is passed through a constriction, wherein said constriction deforms the cell thereby causing a perturbation of the cell such that a tolerogenic factor enters the antigen-presenting cell, thereby generating the tolerogenic antigen-presenting cell. In some embodiments, the tolerogenic factor modulates expression and/or activity of an immunomodulatory agent (such as an immunostimulatory agent *(e.g.,* a costimulatory molecule), an immunosuppressive agent, or an inflammatory or anti-inflammatory molecule).

In certain aspects, the invention provides methods for generating a tolerogenic antigen-presenting cell comprising an antigen, wherein an antigen-presenting cell is passed through a constriction, wherein said constriction deforms the cell thereby causing a perturbation of the cell such that the antigen and a tolerogenic factor enter the antigen-presenting cell, thereby generating the tolerogenic antigen-presenting cell comprising the antigen. In some embodiments, the tolerogenic factor modulates expression and/or activity of an immunomodulatory agent (such as an immunostimulatory agent *(e.g.,* a costimulatory molecule), an immunosuppressive agent, or an inflammatory or anti-inflammatory molecule).

In certain aspects, the invention provides methods for generating an immunosuppressive antigen-presenting cell comprising an antigen, wherein an immunosuppressive antigen-presenting cell is passed through a constriction, wherein said constriction deforms the cell thereby causing a perturbation of the cell such that the antigen enters the immunosuppressive antigen-presenting cell, thereby generating the immunosuppressive antigen-presenting cell comprising the antigen.

In certain aspects, the invention provides methods for generating an immunosuppressive antigen-presenting cell, wherein an antigen-presenting cell is passed through a constriction, wherein said constriction deforms the cell thereby causing a perturbation of the cell such that a tolerogenic factor enters the antigen-presenting cell, thereby generating the immunosuppressive antigen-presenting cell. In some embodiments, the tolerogenic factor modulates expression and/or activity of an immunomodulatory agent (such as an immunostimulatory agent *(e.g.,* a costimulatory molecule), an immunosuppressive agent, or an inflammatory or anti-inflammatory molecule).

In certain aspects, the invention provides methods for generating an immunosuppressive antigen-presenting cell comprising an antigen, wherein an antigen-presenting cell is passed through a constriction, wherein said constriction deforms the cell thereby causing a perturbation of the cell such that the antigen and a tolerogenic factor enter the antigen-presenting cell, thereby generating the immunosuppressive antigen-presenting cell comprising the antigen. In some embodiments, the tolerogenic factor modulates expression and/or activity of an immunomodulatory agent (such as an immunostimulatory agent *(e.g.,* a costimulatory molecule), an immunosuppressive agent, or an inflammatory or anti-inflammatory molecule).

In certain aspects, the invention provides methods for delivering an antigen into a first immune cell *(e.g.,* a tolerogenic immune cell), the method comprising passing a cell suspension comprising the first immune cell through a constriction, wherein said constriction deforms the first immune cell, thereby causing a perturbation of the cell such that the antigen enters the cell, wherein said cell suspension is contacted with the antigen. In some embodiments, a tolerogenic factor is delivered to the first immune cell by the methods disclosed herein, thereby conferring a tolerogenic phenotype to the first immune cell. In some embodiments, the antigen is presented in a tolerogenic manner, e.g., by the first immune cell. In some embodiments, the antigen is presented in a tolerogenic manner by another immune cell, *e.g.,* a second immune cell, such as an immune cell having a tolerogenic phenotype conferred by a property of the first immune cell, or an immune cell having a tolerogenic phenotype independent from the first immune cell. In some embodiments, the antigen is processed and presented by class I MHC by a tolerogenic immune cell *(e.g.,* by the first or second immune cell). In some embodiments, the antigen is processed and presented by class II MHC by a tolerogenic immune cell *(e.g.,* by the first or second immune cell).

In certain aspects, the invention provides methods for delivering a tolerogenic factor that generates a tolerogenic phenotype into a first immune cell, the method comprising passing a cell suspension comprising the first immune cell through a constriction, wherein said constriction deforms the immune cell, thereby causing a perturbation of the cell such that the tolerogenic factor enters the cell, wherein said cell suspension is contacted with the tolerogenic factor, thereby generating a tolerogenic immune cell. In some embodiments, the tolerogenic factor modulates expression and/or activity of an immunomodulatory agent (such as an immunostimulatory agent *(e.g.,* a costimulatory molecule), an immunosuppressive agent, or an inflammatory or anti-inflammatory molecule). In some embodiments, an antigen is endocytosed by the first immune cell or the tolerogenic immune cell. In some embodiments, an antigen is delivered to the first immune cell or the tolerogenic immune cell by any of the methods disclosed herein. In some embodiments, the antigen is presented in a tolerogenic manner, e.g., by the tolerogenic immune cell. In some embodiments, the antigen is presented in a tolerogenic manner by another immune cell, *e.g.,* a second immune cell, *e.g.,* an immune cell having a tolerogenic phenotype conferred by a property of the tolerogenic immune cell, or an immune cell having a tolerogenic phenotype independent from the tolerogenic immune cell. In some embodiments, the antigen is processed and presented by class I MHC by a tolerogenic immune cell *(e.g.,* by the tolerogenic immune cell or the second immune cell). In some embodiments, the antigen is processed and presented by class II MHC by a tolerogenic immune cell *(e.g.,* by the tolerogenic immune cell or the second immune cell).

In certain aspects, the invention provides methods for delivering an antigen and a tolerogenic factor that generates a tolerogenic phenotype into a first immune cell, the method comprising passing a cell suspension comprising the first immune cell through a constriction, wherein said constriction deforms the immune cell, thereby causing a perturbation of the cell such that the antigen and the tolerogenic factor enter the cell, wherein said cell suspension is contacted with the antigen and the tolerogenic factor, thereby generating a tolerogenic immune cell comprising the antigen. In some embodiments, the tolerogenic factor modulates expression and/or activity of an immunomodulatory agent (such as an immunostimulatory agent *(e.g.,* a costimulatory molecule), an immunosuppressive agent, or an inflammatory or anti-inflammatory molecule). In some embodiments, the antigen is presented in a tolerogenic manner, e.g., by the tolerogenic immune cell. In some embodiments, the antigen is presented in a tolerogenic manner by another immune cell, *e.g.,* a second immune cell, *e.g.,* an immune cell having a tolerogenic phenotype conferred by a property of the tolerogenic immune cell, or an immune cell having a tolerogenic phenotype independent from the tolerogenic immune cell. In some embodiments, the antigen is processed and presented by class I MHC by a tolerogenic immune cell *(e.g.,* by the tolerogenic immune cell or the second immune cell). In some embodiments, the antigen is processed and presented by class II MHC by a tolerogenic immune cell *(e.g.,* by the tolerogenic immune cell or the second immune cell).

In certain aspects, the invention provides methods for delivering an antigen into a first immune cell *(e.g.,* an immunosuppressive immune cell), the method comprising passing a cell suspension comprising the first immune cell through a constriction, wherein said constriction deforms the first immune cell, thereby causing a perturbation of the cell such that the antigen enters the cell, wherein said cell suspension is contacted with the antigen. In some embodiments, a tolerogenic factor is delivered to the first immune cell by the methods disclosed herein, thereby conferring an immunosuppressive phenotype to the first immune cell. In some embodiments, the antigen is presented in an immunosuppressive manner, e.g., by the first immune cell. In some embodiments, the antigen is presented in an immunosuppressive manner by another immune cell, *e.g.,* a second immune cell, such as an immune cell having an immunosuppressive phenotype conferred by a property of the first immune cell, or an immune cell having an immunosuppressive phenotype independent from the first immune cell. In some embodiments, the antigen is processed and presented by class I MHC by an immunosuppressive immune cell *(e.g.,* by the first or second immune cell). In some embodiments, the antigen is processed and presented by class II MHC by an immunosuppressive immune cell *(e.g.,* by the first or second immune cell).

In certain aspects, the invention provides methods for delivering a tolerogenic factor that generates an immunosuppressive phenotype into an immune cell, the method comprising passing a cell suspension comprising the immune cell through a constriction, wherein said constriction deforms the immune cell, thereby causing a perturbation of the cell such that the tolerogenic factor that generates an immunosuppressive phenotype enters the cell, wherein said cell suspension is contacted with the tolerogenic factor, thereby generating an immunosuppressive immune cell. In some embodiments, the tolerogenic factor modulates expression and/or activity of an immunomodulatory agent (such as an immunostimulatory agent *(e.g.,* a costimulatory molecule), an immunosuppressive agent, or an inflammatory or anti-inflammatory molecule). In some embodiments, an antigen is endocytosed by the first immune cell or the immunosuppressive immune cell. In some embodiments, an antigen is delivered to the first immune cell or the immunosuppressive immune cell by any of the methods disclosed herein. In some embodiments, the antigen is presented in an immunosuppressive manner, e.g., by the immunosuppressive immune cell. In some embodiments, the antigen is presented in an immunosuppressive manner by another immune cell, *e.g.,* a second immune cell, *e.g.,* an immune cell having an immunosuppressive phenotype conferred by a property of the immunosuppressive immune cell, or an immune cell having a tolerogenic phenotype independent from the immunosuppressive immune cell. In some embodiments, the antigen is processed and presented by class I MHC by an immunosuppressive immune cell *(e.g.,* by the immunosuppressive immune cell or the second immune cell). In some embodiments, the antigen is processed and presented by class II MHC by an immunosuppressive immune cell *(e.g.,* by the immunosuppressive immune cell or the second immune cell).

In certain aspects, the invention provides methods for delivering an antigen and a tolerogenic factor that generates an immunosuppressive phenotype into a first immune cell, the method comprising passing a cell suspension comprising the first immune cell through a constriction, wherein said constriction deforms the immune cell, thereby causing a perturbation of the cell such that the antigen and the tolerogenic factor enter the cell, wherein said cell suspension is contacted with the antigen and the tolerogenic factor, thereby generating an immunosuppressive immune cell comprising the antigen. In some embodiments, the tolerogenic factor modulates expression and/or activity of an immunomodulatory agent (such as an immunostimulatory agent *(e.g.,* a costimulatory molecule), an immunosuppressive agent, or an inflammatory or anti-inflammatory molecule). In some embodiments, the antigen is presented in an immunosuppressive manner, e.g., by the immunosuppressive immune cell. In some embodiments, the antigen is presented in a immunosuppressive manner by another immune cell, *e.g.,* a second immune cell, *e.g.,* an immune cell having an immunosuppressive phenotype conferred by a property of the immunosuppressive immune cell, or an immune cell having an immunosuppressive phenotype independent from the immunosuppressive immune cell. In some embodiments, the antigen is processed and presented by class I MHC by an immunosuppressive immune cell *(e.g.,* by the immunosuppressive immune cell or the second immune cell). In some embodiments, the antigen is processed and presented by class II MHC by an immunosuppressive immune cell *(e.g.,* by the immunosuppressive immune cell or the second immune cell).

In certain aspects, the invention provides methods for suppressing an immune response in an individual, comprising passing a first cell suspension comprising a first immune cell through a constriction, wherein said constriction deforms the cell thereby causing a perturbation of the cell such that an antigen enters the immune cell, passing a second cell suspension comprising a second immune cell through a constriction, wherein said constriction deforms the cell thereby causing a perturbation of the cell such that a tolerogenic factor enters the immune cell, wherein an immunosuppressive immune cell is generated, and introducing the first immune cell and second immune cell into the individual, wherein said antigen is presented in an immunosuppressive manner and suppresses an immune response to the antigen. In some embodiments, the antigen is presented in the first immune cell. In some embodiments, the first immune cell has a tolerogenic phenotype conferred by a property of the second immune cell. In some embodiments, the antigen is presented by the second immune cell. In some embodiments, the antigen is presented by a third immune cell, *e.g.,* an immune cell having a tolerogenic phenotype conferred by a property of the second immune cell. In some embodiments, the third immune cell has a tolerogenic phenotype independent of a property of the second immune cell *(e.g.,* the third immune cell had a tolerogenic phenotype prior to introduction of the second immune cell into the individual). In some embodiments, the tolerogenic factor modulates expression and/or activity of an immunomodulatory agent (such as an immunostimulatory agent *(e.g.,* a costimulatory molecule), an immunosuppressive agent, or an inflammatory or anti-inflammatory molecule). In some embodiments, the first immune cell and second immune cell are introduced simultaneously. In some embodiments, the first immune cell and the second immune cell are combined in a cell suspension prior to introduction to the individual. In some embodiments, the first immune cell and second immune cell are introduced sequentially. In some embodiments, the first immune cell is introduced to the individual before introduction of the second immune cell. In some embodiments, the first immune cell is introduced to the individual more than any of about 1 minute, 5 minutes, 10 minutes, 15 minutes, 30 minutes, 1 hour, 2 hours, 3 hours, 4 hours, 5 hours, 6 hours, 12 hours, or 24 hours before introduction of the second immune cell. In some embodiments, the second immune cell is introduced to the individual before introduction of the first immune cell. In some embodiments, the second immune cell is introduced to the individual more than any of about 1 minute, 5 minutes, 10 minutes, 15 minutes, 30 minutes, 1 hour, 2 hours, 3 hours, 4 hours, 5 hours, 6 hours, 12 hours, or 24 hours before introduction of the first immune cell. In some embodiments, delivery of the tolerogenic factor to the second cell imparts the second cell with a secretory function. For example, the second cell may secrete IL-4, IL-10, IL-13, IFNα, and/or TGFβ, thereby enhancing an immunosuppressive microenvironment. In some embodiments, the immunosuppressive microenvironment allows tolerogenic presentation of antigen by the first cell.

In certain aspects, the invention provides methods for inducing antigen-specific tolerance in an individual, comprising passing a first cell suspension comprising a first immune cell through a constriction, wherein said constriction deforms the cell thereby causing a perturbation of the cell such that an antigen enters the immune cell, passing a second cell suspension comprising a second immune cell through a constriction, wherein said constriction deforms the cell thereby causing a perturbation of the cell such that a tolerogenic factor enters the immune cell, and introducing the first immune cell and second immune cell into the individual, wherein said antigen is presented in a tolerogenic manner and suppresses an immune response to the antigen. In some embodiments, the antigen is presented in the first immune cell. In some embodiments, the first immune cell has a tolerogenic phenotype conferred by a property of the second immune cell. In some embodiments, the antigen is presented by the second immune cell. In some embodiments, the antigen is presented by a third immune cell, *e.g.,* an immune cell having a tolerogenic phenotype conferred by a property of the second immune cell. In some embodiments, the third immune cell has a tolerogenic phenotype independent of a property of the second immune cell *(e.g.,* the third immune cell had a tolerogenic phenotype prior to introduction of the second immune cell into the individual). In some embodiments, the tolerogenic factor modulates expression and/or activity of an immunomodulatory agent (such as an immunostimulatory agent *(e.g.,* a costimulatory molecule), an immunosuppressive agent, or an inflammatory or anti-inflammatory molecule). In some embodiments, the first immune cell and second immune cell are introduced simultaneously. In some embodiments, the first immune cell and second immune cell are introduced sequentially. In some embodiments, the first immune cell and the second immune cell are combined in a cell suspension prior to introduction to the individual. In some embodiments, the first immune cell is introduced to the individual before introduction of the second immune cell. In some embodiments, the first immune cell is introduced to the individual more than any of about 1 minute, 5 minutes, 10 minutes, 15 minutes, 30 minutes, 1 hour, 2 hours, 3 hours, 4 hours, 5 hours, 6 hours, 12 hours, or 24 hours before introduction of the second immune cell. In some embodiments, the second immune cell is introduced to the individual before introduction of the first immune cell. In some embodiments, the second immune cell is introduced to the individual more than any of about 1 minute, 5 minutes, 10 minutes, 15 minutes, 30 minutes, 1 hour, 2 hours, 3 hours, 4 hours, 5 hours, 6 hours, 12 hours, or 24 hours before introduction of the first immune cell. In some embodiments, delivery of the tolerogenic factor to the second cell imparts the second cell with a secretory function. For example, the second cell may secrete IL-4, IL-10, IL-13, IFNα, and/or TGFβ, thereby enhancing an immunosuppressive microenvironment. In some embodiments, the immunosuppressive microenvironment allows tolerogenic presentation of antigen by the first cell.

In certain aspects, the invention provides methods for suppressing an immune response in an individual, the method comprising passing a cell suspension comprising an immune cell through a constriction, wherein said constriction deforms the cell thereby causing a perturbation of the cell such that a compound encoding a nonfunctional cytokine binding protein enters the immune cell, and introducing the immune cell into the individual, wherein said nonfunctional cytokine binding protein is expressed, wherein said nonfunctional cytokine binding protein binds free inflammatory cytokines, thereby suppressing an immune response. For example, the nonfunctional cytokine binding protein may bind and/or sequester free inflammatory cytokines, thereby preventing them from binding to functional cytokine receptors and triggering downstream signaling. In some embodiments, the cytokines are destroyed upon internalization and intracellular processing of the nonfunctional cytokine binding proteins. In some embodiments, the nonfunctional cytokine binding protein comprises a nonfunctional cytokine receptor. In some embodiments, the nonfunctional cytokine receptor lacks cytoplasmic signaling domains. In some embodiments, the nonfunctional cytokine binding protein comprises a proteolytic site that cleaves the target cytokine. For example, the nonfunctional cytokine binding protein may contain an enzyme that cleaves or degrades the cytokine upon binding. In some embodiments, the nonfunctional cytokine binding protein comprises an anti-cytokine antibody. In some embodiments, the nonfunctional cytokine binding protein comprises a B cell receptor. Exemplary cytokines that can be bound by the nonfunctional cytokine receptor include, without limitation, IL-2, IL-6, TNFα, IL-1, and IFNγ.

The immune response is suppressed by at least about 10%. In some embodiments, the immune response is suppressed by at least any of about 15%, about 20%, about 25%, about 30%, about 40%, about 50%, about 60%, about 70%, about 75%, about 80%, about 90%, or about 100%. In some embodiments, the suppressed immune response and/or induced tolerance comprise a decreased T cell response. For example, a decreased T cell response may include, without limitation, decreased T cell activation or proliferation, decreased T cell survival, or decreased cell functionality. In some embodiments, the decreased T cell response comprises decreased T cell activation. In some embodiments, the decreased T cell response comprises decreased T cell survival. In some embodiments, the decreased T cell response comprises decreased T cell proliferation. In some embodiments, the decreased T cell response comprises decreased T cell functionality. For example, decreased T cell functionality can include, without limitation, modulated cytokine secretion, decreased T cell migration to sites of inflammation, and decreased T cell cytotoxic activity. The suppressed immune response comprises decreased production and/or secretion of one or more inflammatory cytokines. In some embodiments, the suppressed immune response and/or induced tolerance comprise increased anti-inflammatory cytokine production and/or secretion. In some embodiments, the suppressed immune response and/or induced tolerance comprise decreased production and/or secretion of one or more inflammatory cytokines selected from interleukin-1 (IL-1), IL-12, and IL-18, tumor necrosis factor (TNF), interferon gamma (IFN-gamma), and granulocyte-macrophage colony stimulating factor (GM-CSF). In some embodiments, the suppressed immune response and/or induced tolerance comprise decreased production and/or secretion of one or more anti-inflammatory cytokines selected from IL-4, IL-10, IL-13, IL-35, IFN-alpha and transforming growth factor-beta (TGFβ). In some embodiments, the suppressed immune response and/or induced tolerance comprises a change in T cell phenotype. For example, the T cell state may change from a proinflammatory phenotype to a regulatory or anti-inflammatory phenotype. In some embodiments, the suppressed immune response and/or induced tolerance comprises uncostimulated activation of a T cell, which may subsequently lead to cell death. In some embodiments, the suppressed immune response and/or induced tolerance comprise an enhanced regulatory T cell (Treg) response. In some embodiments, the suppressed immune response and/or induced tolerance comprise an enhanced regulatory B cell (Breg), tolerogenic monocyte, myeloid derived suppressor cell, or tolerogenic macrophage response. In some embodiments, the suppressed immune response and/or induced tolerance comprise a decreased B cell response. In some embodiments, the decreased B cell response comprises decreased antibody production.

In some embodiments, the suppressed immune response and/or induced tolerance comprise a decreased autoimmune response. For example, the decreased autoimmune response can include, without limitation, a decreased immune response or induced tolerance against an antigen associated with Type I Diabetes, Rheumatoid arthritis, Psoriasis, Multiple Sclerosis, Crohn's disease, or Ulcerative Colitis. In some embodiments, the suppressed immune response and/or induced tolerance comprise a decreased allergic response. For example, the decreased allergic response can include a decreased immune response or induced tolerance against antigens associated with allergic asthma, atopic dermatitis, allergic rhinitis (hay fever), or food allergy. In some embodiments, the antigen is an antigen associated with transplanted tissue. In some embodiments, the suppressed immune response and/or induced tolerance comprises a decreased immune response or induced tolerance against the transplanted tissue. In some embodiments, the antigen is associated with a virus. In some embodiments, the suppressed immune response and/or induced tolerance comprises a decreased pathogenic immune response or induced tolerance to the virus. For example, the pathogenic immune response can include the cytokine storm generated by certain viral infections. A cytokine storm is a potentially fatal immune reaction consisting of a positive feedback loop between cytokines and white blood cells.

In some embodiments, the suppressed immune response comprises a decreased immune response against a therapeutic agent. In some embodiments, the therapeutic agent is a clotting factor. Exemplary clotting factors include, without limitation, Factor VIII and Factor IX. In some embodiments, the therapeutic agent is an antibody. Exemplary therapeutic antibodies include, without limitation, anti-TNFα, anti-VEGF, anti-CD3, anti-CD20, anti-IL-2R, anti-Her2, anti-RSVF, anti-CEA, anti-IL-1beta, anti-CD15, anti-myosin, anti-PSMA, anti-40 kDa glycoprotein, anti-CD33, anti-CD52, anti-IgE, anti-CD1 1a, anti-EGFR, anti-C5, anti-alpha-4 integrin, anti-IL-12/IL-23, anti-IL-6R, and anti-RANKL. In some embodiments, the therapeutic agent is a growth factor. Exemplary therapeutic growth factors include, without limitation, Erythropoietin (EPO) and megakaryocyte differentiation and growth factor (MDGF). In some embodiments, the therapeutic agent is a hormone. Exemplary therapeutic hormones include, without limitation, insulin, human growth hormone, and follicle stimulating hormone. In some embodiments, the therapeutic agent is a recombinant cytokine. Exemplary therapeutic recombinant cytokines include, without limitation, IFNβ, IFNα, and GM-CSF. In some embodiments, the suppressed immune response comprises a decreased immune response against a therapeutic vehicle. In some embodiments, the therapeutic vehicle is a virus, such as an adenovirus, adeno-associated virus, baculovirus, herpes virus, or retrovirus used for gene therapy. In some embodiments, the therapeutic vehicle is a lipid-based vehicle, *e.g.,* a liposome. In some embodiments, the therapeutic vehicle is a nanoparticle.

### IV. MICROFLUIDIC CHANNELS TO PROVIDE CELL-DEFORMING CONSTRICTIONS

In some embodiments, the invention provides methods for suppressing an immune response or inducing tolerance by passing a cell suspension through a constriction, wherein the constriction deforms the immune cell thereby causing a perturbation of the cell such that an antigen or tolerogenic factor enters the cell, wherein the constriction is contained within a microfluidic channel. In some embodiments, multiple constrictions can be placed in parallel and/or in series within the microfluidic channel. Exemplary microfluidic channels containing cell-deforming constrictions for use in the methods disclosed herein are described in WO2013059343. Exemplary surfaces having pores for use in the methods disclosed herein are described in U.S. Provisional Application 62/214,820, filed 09/04/2015.

In some embodiments, the microfluidic channel includes a lumen and is configured such that a cell suspended in a buffer can pass through, wherein the microfluidic channel includes a constriction. The microfluidic channel can be made of any one of a number of materials, including silicon, metal (e.g., stainless steel), plastic (e.g., polystyrene), ceramics, glass, crystalline substrates, amorphous substrates, or polymers (e.g., Poly-methyl methacrylate (PMMA), PDMS, Cyclic Olefin Copolymer (COC), etc.). Fabrication of the microfluidic channel can be performed by any method known in the art, including dry etching, wet etching, photolithography, injection molding, laser ablation, or SU-8 masks.

In some embodiments, the constriction within the microfluidic channel includes an entrance portion, a centerpoint, and an exit portion. In some embodiments, the length, depth, and width of the constriction within the microfluidic channel can vary. In some embodiments, the diameter of the constriction within the microfluidic channel is a function of the diameter of the cell or cluster of cells. In some embodiments, the diameter of the constriction within the microfluidic channel is about 20%, to about 99% of the diameter of the cell. In some embodiments, the constriction size is about 20%, about 30%, about 40%, about 50%, about 60%, about 70%, about 80%, about 90%, or about 99% of the cell diameter. In some embodiments, the constriction size is about 20%, about 30%, about 40%, about 50%, about 60%, about 70%, about 80%, about 90%, or about 99% of the minimum cross-sectional distance of the cell. In some embodiments, the channel comprises a constriction width of between about 2 µm and about 10 µm or any width or range of widths therebetween. For example, the constriction width can be any one of about 2µm, about 3µm, about 4µm, about 5µm, about 6µm, or about 7µm. In some embodiments, the channel comprises a constriction length of about 10 µm and a constriction width of about 4 µm. The cross-section of the channel, the entrance portion, the centerpoint, and the exit portion can also vary. For example, the cross-sections can be circular, elliptical, an elongated slit, square, hexagonal, or triangular in shape. The entrance portion defines a constriction angle, wherein the constriction angle is optimized to reduce clogging of the channel and optimized for enhanced delivery of a compound into the cell. The angle of the exit portion can vary as well. For example, the angle of the exit portion is configured to reduce the likelihood of turbulence that can result in non-laminar flow. In some embodiments, the walls of the entrance portion and/or the exit portion are linear. In other embodiments, the walls of the entrance portion and/or the exit portion are curved.

### V. SURFACE HAVING PORES TO PROVIDE CELL DEFORMING CONSTRICTIONS

In some embodiments, the invention provides methods for suppressing an immune response or inducing tolerance by passing a cell suspension through a constriction, wherein the constriction deforms the immune cell thereby causing a perturbation of the cell such that an antigen and/or tolerogenic factor enters the cell, wherein the constriction is a pore or contained within a pore. In some embodiments, the pore is contained in a surface. Exemplary surfaces having pores for use in the methods disclosed herein are described in U.S. Provisional Application 62/214,820, filed 09/04/2015.

The surfaces as disclosed herein can be made of any one of a number of materials and take any one of a number of forms. In some embodiments, the surface is a filter. In some embodiments, the surface is a membrane. In some embodiments, the filter is a tangential flow filter. In some embodiments, the surface is a sponge or sponge-like matrix. In some embodiments, the surface is a matrix.

In some embodiments the surface is a tortuous path surface. In some embodiments, the tortuous path surface comprises cellulose acetate. In some embodiments, the surface comprises a material selected from, without limitation, synthetic or natural polymers, polycarbonate, silicon, glass, metal, alloy, cellulose nitrate, silver, cellulose acetate, nylon, polyester, polyethersulfone, Polyacrylonitrile (PAN), polypropylene, PVDF, polytetrafluorethylene, mixed cellulose ester, porcelain, and ceramic.

The surface disclosed herein can have any shape known in the art; e.g. a 3-dimensional shape. The 2-dimensional shape of the surface can be, without limitation, circular, elliptical, round, square, star-shaped, triangular, polygonal, pentagonal, hexagonal, heptagonal, or octagonal. In some embodiments, the surface is round in shape. In some embodiments, the surface 3-dimensional shape is cylindrical, conical, or cuboidal.

The surface can have various cross-sectional widths and thicknesses. In some embodiments, the surface cross-sectional width is between about 1mm and about 1m or any cross-sectional width or range of cross-sectional widths therebetween. In some embodiments, the surface has a defined thickness. In some embodiments, the surface thickness is uniform. In some embodiments, the surface thickness is variable. For example, in some embodiments, portions of the surface are thicker or thinner than other portions of the surface. In some embodiments, the surface thickness varies by about 1% to about 90% or any percentage or range of percentages therebetween. In some embodiments, the surface is between about 0.01µm to about 5mm thick or any thickness or range of thicknesses therebetween.

In some embodiments, the constriction is a pore or contained within a pore. The cross-sectional width of the pores is related to the type of cell to be treated. In some embodiments, the pore size is a function of the diameter of the cell or cluster of cells to be treated. In some embodiments, the pore size is such that a cell is perturbed upon passing through the pore. In some embodiments, the pore size is less than the diameter of the cell. In some embodiments, the pore size is about 10% to about 99% of the diameter of the cell. In some embodiments, the pore size is about 10%, about 15%, about 20%, about 30%, about 40%, about 50%, about 60%, about 70%, about 80%, about 90%, or about 99% of the cell diameter. Optimal pore size or pore cross-sectional width can vary based upon the application and/or cell type. In some embodiments, the pore size is about 2 µm to about 14 µm. In some embodiments, the pore size is about 2µm, about 3µm, about 4µm, about 5µm, about 8µm, about 10µm, about 12µm, or about 14µm. In some embodiments, the cross-sectional width is about 2 µm to about 14 µm. In some embodiments, the pore cross-sectional is about 2µm, about 3µm, about 4µm, about 5µm, about 8µm, about 10µm, about 12µm, or about 14µm.

The entrances and exits of the pore passage may have a variety of angles. The pore angle can be selected to minimize clogging of the pore while cells are passing through. In some embodiments, the flow rate through the surface is between about 0.001 mL/cm²/sec to about 100 L/cm²/sec or any rate or range of rates therebetween. For example, the angle of the entrance or exit portion can be between about 0 and about 90 degrees. In some embodiments, the entrance or exit portion can be greater than 90 degrees. In some embodiments, the pores have identical entrance and exit angles. In some embodiments, the pores have different entrance and exit angles. In some embodiments, the pore edge is smooth, e.g. rounded or curved. A smooth pore edge has a continuous, flat, and even surface without bumps, ridges, or uneven parts. In some embodiments, the pore edge is sharp. A sharp pore edge has a thin edge that is pointed or at an acute angle. In some embodiments, the pore passage is straight. A straight pore passage does not contain curves, bends, angles, or other irregularities. In some embodiments, the pore passage is curved. A curved pore passage is bent or deviates from a straight line. In some embodiments, the pore passage has multiple curves, e.g. about 2, 3, 4, 5, 6, 7, 8, 9, 10 or more curves.

The pores can have any shape known in the art, including a 2-dimensional or 3-dimensional shape. The pore shape (e.g., the cross-sectional shape) can be, without limitation, circular, elliptical, round, square, star-shaped, triangular, polygonal, pentagonal, hexagonal, heptagonal, and octagonal. In some embodiments, the cross-section of the pore is round in shape. In some embodiments, the 3-dimensional shape of the pore is cylindrical or conical. In some embodiments, the pore has a fluted entrance and exit shape. In some embodiments, the pore shape is homogenous (i.e. consistent or regular) among pores within a given surface. In some embodiments, the pore shape is heterogeneous (i.e. mixed or varied) among pores within a given surface.

The surfaces described herein can have a range of total pore numbers. In some embodiments, the pores encompass about 10% to about 80% of the total surface area. In some embodiments, the surface contains about 1.0x10⁵ to about 1.0x10³⁰ total pores or any number or range of numbers therebetween. In some embodiments, the surface comprises between about 10 and about 1.0x10¹⁵ pores per mm² surface area.

The pores can be distributed in numerous ways within a given surface. In some embodiments, the pores are distributed in parallel within a given surface. In one such example, the pores are distributed side-by-side in the same direction and are the same distance apart within a given surface. In some embodiments, the pore distribution is ordered or homogeneous. In one such example, the pores are distributed in a regular, systematic pattern or are the same distance apart within a given surface. In some embodiments, the pore distribution is random or heterogeneous. In one such example, the pores are distributed in an irregular, disordered pattern or are different distances apart within a given surface. In some embodiments, multiple surfaces are distributed in series. The multiple surfaces can be homogeneous or heterogeneous in surface size, shape, and/or roughness. The multiple surfaces can further contain pores with homogeneous or heterogeneous pore size, shape, and/or number, thereby enabling the simultaneous delivery of a range of compounds into different cell types.

In some embodiments, an individual pore has a uniform width dimension (i.e. constant width along the length of the pore passage). In some embodiments, an individual pore has a variable width (i.e. increasing or decreasing width along the length of the pore passage). In some embodiments, pores within a given surface have the same individual pore depths. In some embodiments, pores within a given surface have different individual pore depths. In some embodiments, the pores are immediately adjacent to each other. In some embodiments, the pores are separated from each other by a distance. In some embodiments, the pores are separated from each other by a distance of about 0.001µm to about 30mm or any distance or range of distances therebetween.

In some embodiments, the surface is coated with a material. The material can be selected from any material known in the art, including, without limitation, Teflon, an adhesive coating, surfactants, proteins, adhesion molecules, antibodies, anticoagulants, factors that modulate cellular function, nucleic acids, lipids, carbohydrates, or transmembrane proteins. In some embodiments, the surface is coated with polyvinylpyrrolidone. In some embodiments, the material is covalently attached to the surface. In some embodiments, the material is non`-covalently attached to the surface. In some embodiments, the surface molecules are released at the cells pass through the pores.

In some embodiments, the surface has modified chemical properties. In some embodiments, the surface is polar. In some embodiments, the surface is hydrophilic. In some embodiments, the surface is non-polar. In some embodiments, the surface is hydrophobic. In some embodiments, the surface is charged. In some embodiments, the surface is positively and/or negatively charged. In some embodiments, the surface can be positively charged in some regions and negatively charged in other regions. In some embodiments, the surface has an overall positive or overall negative charge. In some embodiments, the surface can be any one of smooth, electropolished, rough, or plasma treated. In some embodiments, the surface comprises a zwitterion or dipolar compound. In some embodiments, the surface is plasma treated.

In some embodiments, the surface is contained within a larger module. In some embodiments, the surface is contained within a syringe, such as a plastic or glass syringe. In some embodiments, the surface is contained within a plastic filter holder. In some embodiments, the surface is contained within a pipette tip.

### VI. CELL PERTURBATIONS

In some embodiments, the invention provides methods for suppressing an immune response or inducing tolerance by passing a cell suspension through a constriction, wherein the constriction deforms the immune cell thereby causing a perturbation of the cell such that an antigen or tolerogenic factor enters the cell, wherein the perturbation in the cell is a breach in the cell that allows material from outside the cell to move into the cell (e.g., a hole, tear, cavity, aperture, pore, break, gap, perforation). The deformation can be caused by, for example, pressure induced by mechanical strain and/or shear forces. In some embodiments, the perturbation is a perturbation within the cell membrane. In some embodiments, the perturbation is transient. In some embodiments, the cell perturbation lasts from about 1.0x10⁻⁹ seconds to about 2 hours, or any time or range of times therebetween. In some embodiments, the cell perturbation lasts for about 1.0x10⁻⁹second to about 1 second, about 1 second to about 1 minute, or about 1 minute to about 1 hour. In some embodiments, the cell perturbation lasts for between any one of about 1.0x10⁻⁹ to about 1.0x10⁻¹, about 1.0x10⁻⁹ to about 1.0x10⁻², about 1.0x10⁻⁹ to about 1.0x10⁻³, about 1.0x10⁻⁹ to about 1.0x10⁻⁴, about 1.0x10⁻⁹ to about 1.0x10⁻⁵, about 1.0x10⁻⁹ to about 1.0x10⁻⁶, about 1.0x10⁻⁹ to about 1.0x10⁻⁷, or about 1.0x10⁻⁹ to about 1.0x10⁻⁸ seconds. In some embodiment, the cell perturbation lasts for any one of about 1.0x10⁻⁸ to about 1.0x10⁻¹, about 1.0x10⁻⁷ to about 1.0x10⁻¹, about 1.0x10⁻⁶ to about 1.0x10⁻¹, about 1.0x10⁻⁵ to about 1.0x10⁻¹, about 1.0x10⁻⁴ to about 1.0x10⁻¹, about 1.0x10⁻³ to about 1.0x10⁻¹, or about 1.0x10⁻² to about 1.0x10⁻¹ seconds. The cell perturbations (e.g., pores or holes) created by the methods described herein are not formed as a result of assembly of protein subunits to form a multimeric pore structure such as that created by complement or bacterial hemolysins.

As the cell passes through the constriction, the constriction temporarily imparts injury to the cell membrane that causes passive diffusion of material through the perturbation. In some embodiments, the cell is only deformed for a brief period of time, on the order of 100 µs to minimize the chance of activating apoptotic pathways through cell signaling mechanisms, although other durations are possible (e.g., ranging from nanoseconds to hours). In some embodiments, the cell is deformed for about 1.0 x10⁻⁹ seconds to about 2 hours, or any time or range of times therebetween. In some embodiments, the cell is deformed for about 1.0x10⁻⁹ second to about 1 second, about 1 second to about 1 minute, or about 1 minute to about 1 hour. In some embodiments, the cell is deformed for between any one of about 1.0x10⁻⁹ to about 1.0x10⁻¹, about 1.0x10⁻⁹ to about 1.0x10⁻², about 1.0x10⁻⁹ to about 1.0x10⁻³, about 1.0x10⁻⁹ to about 1.0x10⁻⁴, about 1.0x10⁻⁹ to about 1.0x10⁻⁵, about 1.0x10⁻⁹ to about 1.0x10⁻⁶, about 1.0x10⁻⁹ to about 1.0x10⁻⁷, or about 1.0x10⁻⁹ to about 1.0x10⁻⁸ seconds. In some embodiment, the cell is deformed for any one of about 1.0x10⁻⁸ to about 1.0x10⁻¹, about 1.0x10⁻⁷ to about 1.0x10⁻¹, about 1.0x10⁻⁶ to about 1.0x10⁻¹, about 1.0x10⁻⁵ to about 1.0x10⁻¹, about 1.0x10⁻⁴ to about 1.0x10⁻¹, about 1.0x10⁻³ to about 1.0x10⁻¹, or about 1.0x10⁻² to about 1.0x10⁻¹ seconds. In some embodiments, deforming the cell includes deforming the cell for a time ranging from, without limitation, about 1 µs to at least about 750 µs, e.g., at least about 1µs, 10 µs, 50 µs, 100 µs, 500 µs, or 750 µs .

In some embodiments, the passage of the compound into the cell occurs simultaneously with the cell passing through the constriction and/or the perturbation of the cell. In some embodiments, passage of the compound into the cell occurs after the cell passes through the constriction. In some embodiments, passage of the compound into the cell occurs on the order of minutes after the cell passes through the constriction. In some embodiments, the passage of the compound into the cell occurs from about 1.0x10⁻² seconds to at least about 30 minutes after the cell passes through the constriction. For example, the passage of the compound into the cell occurs from about 1.0x10⁻² seconds to about 1 second, about 1 second to about 1 minute, or about 1 minute to about 30 minutes after the cell passes through the constriction. In some embodiments, the passage of the compound into the cell occurs about 1.0x10⁻² seconds to about 10 minutes, about 1.0x10⁻² seconds to about 5 minutes, about 1.0x10⁻² seconds to about 1 minute, about 1.0x10⁻² seconds to about 50 seconds, about 1.0x10⁻² seconds to about 10 seconds, about 1.0x10⁻² seconds to about 1 second, or about 1.0x10⁻² seconds to about 0.1 second after the cell passes through the constriction. In some embodiments, the passage of the compound into the cell occurs about 1.0x10⁻¹ seconds to about 10 minutes, about 1 second to about 10 minutes, about 10 seconds to about 10 minute, about 50 seconds to about 10 minutes, about 1 minute to about 10 minutes, or about 5 minutes to about 10 minutes after the cell passes through the constriction. In some embodiments, a perturbation in the cell after it passes through the constriction is corrected within the order of about five minutes after the cell passes through the constriction.

In some embodiments, the cell viability after passing through a constriction is about 5% to about 100%. In some embodiments, the cell viability after passing through the constriction is at least about 5%, 10%, 20%, 30%, 40%, 50%, 60%, 70%, 75%, 80%, 85%, 90%, 95%, or 99%. In some embodiments, the cell viability is measured from about 1.0x10⁻² seconds to at least about 10 days after the cell passes through the constriction. For example, the cell viability is measured from about 1.0x10⁻² seconds to about 1 second, about 1 second to about 1 minute, about 1 minute to about 30 minutes, or about 30 minutes to about 2 hours after the cell passes through the constriction. In some embodiments, the cell viability is measured about 1.0x10⁻² seconds to about 2 hours, about 1.0x10⁻² seconds to about 1 hour, about 1.0x10⁻² seconds to about 30 minutes, about 1.0x10⁻² seconds to about 1 minute, about 1.0x10⁻² seconds to about 30 seconds, about 1.0x10⁻² seconds to about 1 second, or about 1.0x10⁻² seconds to about 0.1 second after the cell passes through the constriction. In some embodiments, the cell viability is measured about 1.5 hours to about 2 hours, about 1 hour to about 2 hours, about 30 minutes to about 2 hours, about 15 minutes to about 2 hours, about 1 minute to about 2 hours, about 30 seconds to about 2 hours, or about 1 second to about 2 hours after the cell passes through the constriction. In some embodiments, the cell viability is measured about 2 hours to about 5 hours, about 5 hours to about 12 hours, about 12 hours to about 24 hours, or about 24 hours to about 10 days after the cell passes through the constriction.

### VII. DELIVERY PARAMETERS

A number of parameters may influence the delivery of a compound to a cell for suppressing an immune response or inducing tolerance by the methods described herein. In some embodiments, the cell suspension is contacted with the compound before, concurrently, or after passing through the constriction. The cell may pass through the constriction suspended in a solution that includes the compound to deliver, although the compound can be added to the cell suspension after the cells pass through the constriction. In some embodiments, the compound to be delivered is coated on the constriction.

Examples of parameters that may influence the delivery of the compound into the cell include, but are not limited to, the dimensions of the constriction, the entrance angle of the constriction, the surface properties of the constrictions (e.g., roughness, chemical modification, hydrophilic, hydrophobic, etc.), the operating flow speeds (e.g., cell transit time through the constriction), the cell concentration, the concentration of the compound in the cell suspension, and the amount of time that the cell recovers or incubates after passing through the constrictions can affect the passage of the delivered compound into the cell. Additional parameters influencing the delivery of the compound into the cell can include the velocity of the cell in the constriction, the shear rate in the constriction, the viscosity of the cell suspension, the velocity component that is perpendicular to flow velocity, and time in the constriction. Such parameters can be designed to control delivery of the compound. In some embodiments, the cell concentration ranges from about 10 to at least about 10¹² cells/ml or any concentration or range of concentrations therebetween. In some embodiments, delivery compound concentrations can range from about 10 ng/ml to about 1g/mL or any concentration or range of concentrations therebetween. In some embodiments, delivery compound concentrations can range from about 1pM to at least about 2M or any concentration or range of concentrations therebetween.

The temperature used in the methods of the present disclosure can be adjusted to affect compound delivery and cell viability. In some embodiments, the method is performed between about -5°C and about 45°C. For example, the methods can be carried out at room temperature (e.g., about 20°C), physiological temperature (e.g., about 37°C), higher than physiological temperature (e.g., greater than about 37°C to 45°C or more), or reduced temperature (e.g., about -5°C to about 4°C), or temperatures between these exemplary temperatures.

Various methods can be utilized to drive the cells through the constrictions. For example, pressure can be applied by a pump on the entrance side (e.g., gas cylinder, or compressor), a vacuum can be applied by a vacuum pump on the exit side, capillary action can be applied through a tube, and/or the system can be gravity fed. Displacement based flow systems can also be used (e.g., syringe pump, peristaltic pump, manual syringe or pipette, pistons, etc.). In some embodiments, the cells are passed through the constrictions by positive pressure or negative pressure. In some embodiments, the cells are passed through the constrictions by constant pressure or variable pressure. In some embodiments, pressure is applied using a syringe. In some embodiments, pressure is applied using a pump. In some embodiments, the pump is a peristaltic pump or a diaphragm pump. In some embodiments, pressure is applied using a vacuum. In some embodiments, the cells are passed through the constrictions by g-force. In some embodiments, the cells are passed through the constrictions by centrifugal force. In some embodiments, the cells are passed through the constrictions by capillary pressure.

In some embodiments, fluid flow directs the cells through the constrictions. In some embodiments, the fluid flow is turbulent flow prior to the cells passing through the constriction. Turbulent flow is a fluid flow in which the velocity at a given point varies erratically in magnitude and direction. In some embodiments, the fluid flow through the constriction is laminar flow. Laminar flow involves uninterrupted flow in a fluid near a solid boundary in which the direction of flow at every point remains constant. In some embodiments, the fluid flow is turbulent flow after the cells pass through the constriction. The velocity at which the cells pass through the constrictions can be varied. In some embodiments, the cells pass through the constrictions at a uniform cell speed. In some embodiments, the cells pass through the constrictions at a fluctuating cell speed.

In other embodiments, a combination treatment is used to suppress an immune response or induce tolerance by passing a cell suspension through a constriction, wherein the constriction deforms the immune cell thereby causing a perturbation of the cell such that an antigen or tolerogenic factor enters the cell, e.g., the methods described herein followed by exposure to an electric field downstream of the constriction. In some embodiments, the cell is passed through an electric field generated by at least one electrode after passing through the constriction. In some embodiments, the electric field assists in delivery of compounds to a second location inside the cell such as the cell nucleus. For example, the combination of a cell-deforming constriction and an electric field delivers a plasmid encoding an antibody into the cell (e.g., the cell nucleus), resulting in the de novo production of antibody. In some embodiments, one or more electrodes are in proximity to the cell-deforming constriction to generate an electric field. In some embodiments, the electric field is between about 0.1kV/m to about 100MV/m, or any number or range of numbers therebetween. In some embodiments, an integrated circuit is used to provide an electrical signal to drive the electrodes. In some embodiments, the cells are exposed to the electric field for a pulse width of between about 1ns to about 1s and a period of between about 100ns to about 10s or any time or range of times therebetween.

### VIII. CELL SUSPENSIONS FOR DELIVERY TO IMMUNE CELLS

The cell suspension may be a mixed or purified population of cells. In some embodiments, the cell suspension is a mixed cell population, such as whole blood. In some embodiments, the cell suspension is a purified cell population, such as a purified population of immune cells.

The composition of the cell suspension (e.g., osmolarity, salt concentration, serum content, cell concentration, pH, etc.) can impact delivery of the compound for suppressing an immune response or inducing tolerance. In some embodiments, the suspension comprises whole blood. Alternatively, the cell suspension is a mixture of cells in a physiological saline solution or physiological medium other than blood. In some embodiments, the cell suspension comprises an aqueous solution. In some embodiments, the aqueous solution comprises cell culture medium, PBS, salts, sugars, growth factors, animal derived products, bulking materials, surfactants, lubricants, vitamins, amino acids, proteins, cell cycle inhibitors, and/or an agent that impacts actin polymerization. In some embodiments, the cell culture medium is DMEM, Opti-MEM^{®}, IMDM, or RPMI. Additionally, solution buffer can include one or more lubricants (pluronics or other surfactants) that can be designed, for example, to reduce or eliminate clogging of the surface and improve cell viability. Exemplary surfactants include, without limitation, poloxamer, polysorbates, sugars or sugar alcohols such as mannitol, sorbitol, animal derived serum, and albumin protein.

In some configurations with certain types of cells, the cells can be incubated in one or more solutions that aid in the delivery of the compound to the interior of the cell. In some embodiments, the aqueous solution comprises an agent that impacts actin polymerization. In some embodiments, the agent that impacts actin polymerization is Latrunculin A, Cytochalasin, and/or Colchicine. For example, the cells can be incubated in a depolymerization solution such as Lantrunculin A (0.1µg/ml) for 1 hour prior to delivery to depolymerize the actin cytoskeleton. As an additional example, the cells can be incubated in 10µM Colchicine (Sigma) for 2 hours prior to delivery to depolymerize the microtubule network.

In some embodiments, the cell population is enriched prior to use in the disclosed methods. For example, cells are obtained from a bodily fluid, e.g., peripheral blood, and optionally enriched or purified to concentrate B cells. Cells may be enriched by any methods known in the art, including without limitation, magnetic cell separation, fluorescent activated cell sorting (FACS), or density gradient centrifugation.

The viscosity of the cell suspension can also impact the methods disclosed herein. In some embodiments, the viscosity of the cell suspension ranges from about 8.9x10-4Pa.s to about 4.0x10-3Pa.s or any value or range of values therebetween. In some embodiments, the viscosity ranges between any one of about 8.9x10-4 Pa.s to about 4.0 x10-3 Pa.s, about 8.9x10-4 Pa.s to about 3.0 x10-3 Pa.s, about 8.9x10-4 Pa.s to about 2.0 x10-3 Pa.s, or about 8.9x10-3 Pa.s to about 1.0 x10-3 Pa.s. In some embodiments, the viscosity ranges between any one of about 0.89 cP to about 4.0 cP, about 0.89 cP to about 3.0 cP, about 0.89 cP to about 2.0 cP, or about 0.89 cP to about 1.0 cP. In some embodiments, a shear thinning effect is observed, in which the viscosity of the cell suspension decreases under conditions of shear strain. Viscosity can be measured by any method known in the art, including without limitation, viscometers, such as a glass capillary viscometer, or rheometers. A viscometer measures viscosity under one flow condition, while a rheometer is used to measure viscosities which vary with flow conditions. In some embodiments, the viscosity is measured for a shear thinning solution such as blood. In some embodiments, the viscosity is measured between about -5°C and about 45°C. For example, the viscosity is measured at room temperature (e.g., about 20°C), physiological temperature (e.g., about 37°C), higher than physiological temperature (e.g., greater than about 37°C to 45°C or more), reduced temperature (e.g., about -5°C to about 4°C), or temperatures between these exemplary temperatures.

### IX. ANTIGENS AND TOLEROGENIC FACTORS TO SUPPRESS AN IMMUNE RESPONSE OR INDUCE TOLERANCE

The invention provides delivery of antigens to suppress an immune response or induce tolerance, wherein the antigen is delivered to the cell by any of the methods described herein. In some embodiments, the antigen is a single antigen. In some embodiments, the antigen is a mixture of antigens. An antigen is a substance that stimulates a specific immune response, such as a cell or antibody-mediated immune response. Antigens bind to receptors expressed by immune cells, such as T cell receptors (TCRs), which are specific to a particular antigen. Antigen-receptor binding subsequently triggers intracellular signaling pathways that lead to downstream immune effector pathways, such as cell activation, cytokine production, cell migration, cytotoxic factor secretion, and antibody production.

In some embodiments, the antigen is a polypeptide antigen. In some embodiments, the compound comprises a disease-associated antigen. In some embodiments, antigens are derived from foreign sources, such as bacteria, fungi, viruses, or allergens. In some embodiments, antigens are derived from internal sources, such as self-proteins (i.e. self-antigens). In some embodiments, the antigen is in a cell lysate. Self-antigens are antigens present on or in an organism's own cells. Self-antigens do not normally stimulate an immune response, but may in the context of autoimmune diseases, such as Type I Diabetes or Rheumatoid Arthritis. In some embodiments, the antigen is a therapeutic agent. In some embodiments, the antigen is a therapeutic polypeptide, or a fragment of a therapeutic polypeptide. In some embodiments, the therapeutic agent is a clotting factor. Exemplary clotting factors include, without limitation, Factor VIII and Factor IX. For example, the antigen may be a Factor VIII protein used in the treatment of hemophilia. In some embodiments, the therapeutic agent is an antibody. Exemplary therapeutic antibodies include, without limitation, anti-TNFα, anti-VEGF, anti-CD3, anti-CD20, anti-IL-2R, anti-Her2, anti-RSVF, anti-CEA, anti-IL-1beta, anti-CD15, anti-myosin, anti-PSMA, anti-40 kDa glycoprotein, anti-CD33, anti-CD52, anti-IgE, anti-CD11a, anti-EGFR, anti-C5, anti-alpha-4 integrin, anti-IL-12/IL-23, anti-IL-6R, and anti-RANKL. In some embodiments, the therapeutic agent is a growth factor. Exemplary therapeutic growth factors include, without limitation, Erythropoietin (EPO) and megakaryocyte differentiation and growth factor (MDGF). In some embodiments, the therapeutic agent is a hormone. Exemplary therapeutic hormones include, without limitation, insulin, human growth hormone, and follicle stimulating hormone. In some embodiments, the therapeutic agent is a recombinant cytokine. Exemplary therapeutic recombinant cytokines include, without limitation, IFNβ, IFNα, and GM-CSF.

In some embodiments, the antigen is an allograft transplantation antigen. An allograft transplant is a transfer of cells, tissues, or organs, to a recipient from a genetically non-identical donor of the same species. In some embodiments, the antigen is a modified antigen. For example, antigens may be fused with therapeutic agents or targeting peptides. In some embodiments, the modified antigen is fused with a polypeptide. In some embodiments, the modified antigen is fused with a lipid. In some embodiments, the antigen is a non-protein antigen, such as a lipid, glycolipid, or polysaccharide. In some embodiments, the antigen is a whole microorganism, such as an intact bacterium.

In some embodiments, the antigen is associated with a virus. In some embodiments, the antigen is a viral antigen. Exemplary viral antigens include SARS-CoV antigens and influenza antigens. In some embodiments, the compound comprises cell lysate from tissue infected with an unknown pathogen. In some embodiments, the antigen is a therapeutic vehicle. In some embodiments, the therapeutic vehicle is a virus, such as an adenovirus, adeno-associated virus, baculovirus, herpes virus, or retrovirus used for gene therapy. In some embodiments, the therapeutic vehicle is a liposome. In some embodiments, the therapeutic vehicle is a nanoparticle.

In some embodiments, the antigen is associated with a microorganism; for example a bacterium. In some embodiments, the suppressed immune response and/or induced tolerance comprises a decreased pathogenic immune response or induced tolerance to the microorganism; for example, a bacterium. In some embodiments, the microorganism is part of the microbiome of the individual. In some embodiments, the microorganism may benefit the microbiome of the individual.

In certain aspects, the invention provides methods for delivering an antigen into an immune cell, the method comprising passing a cell suspension comprising the immune cell through a constriction, wherein said constriction deforms the immune cell, thereby causing a perturbation of the cell such that the antigen enters the cell, wherein said cell suspension is contacted with the antigen. In some embodiments, processing and presentation of the antigen in a tolerogenic environment suppresses an immune response to the antigen. In some embodiments, processing and presentation of said antigen in a tolerogenic environment induces tolerance to the antigen. In some embodiments, the antigen is delivered to the immune cell in vitro, ex vivo, or in vivo.

In some embodiments the invention provides delivery of tolerogenic factors to suppress an immune response or induce tolerance, wherein the tolerogenic factor is delivered to the cell by any of the methods described herein. In some embodiments, the tolerogenic factor enhances suppression of an immune response to an antigen or enhances the induction of tolerance to an antigen. For example, the tolerogenic factor may promote tolerogenic presentation of the antigen by an antigen-presenting cell. In some embodiments, the tolerogenic factor is introduced simultaneously with the antigen. In some embodiments, the tolerogenic factor and antigen are introduced sequentially.

In certain aspects, the invention provides methods for generating an immunosuppressive antigen-presenting immune cell comprising an antigen, wherein the immunosuppressive immune cell is passed through a constriction, wherein said constriction deforms the cell thereby causing a perturbation of the cell such that an antigen enters the immune cell, thereby generating the immunosuppressive immune cell comprising the antigen.

In some embodiments, the tolerogenic factor modulates expression and/or activity of an immunomodulatory agent (such as an immunostimulatory agent (*e.g.,* a costimulatory molecule), an immunosuppressive agent, or an inflammatory or anti-inflammatory molecule). In some embodiments, the tolerogenic factor inhibits expression and/or activity of an immunostimulatory agent (*e.g.,* a costimulatory molecule), enhances expression and/or activity of an immunosuppressive molecule, inhibits expression and/or activity of an inflammatory molecule, and/or enhances expression and/or activity of an anti-inflammatory molecule. In some embodiments, the tolerogenic factor inhibits the activity of a costimulatory molecule. Interaction between costimulatory molecules and their ligands is important to sustain and integrate TCR signaling to stimulate optimal T cell proliferation and differentiation. In some embodiments, the tolerogenic factor decreases expression of a costimulatory molecule. Exemplary costimulatory molecules expressed on antigen-presenting cells include, without limitation, CD40, CD80, CD86, CD54, CD83, CD79, or ICOS Ligand. In some embodiments, the costimulatory molecule is CD80 or CD86. In some embodiments, the tolerogenic factor inhibits the expression of a nucleic acid that expresses or modulates expression of the costimulatory molecule. In some embodiments, the tolerogenic factor deletes a nucleic acid that expresses or modulates expression of the costimulatory molecule. In some embodiments, deletion of the nucleic acid that expresses or modulates expression of the costimulatory molecule is achieved via gene editing. In some embodiments, the tolerogenic factor inhibits the costimulatory molecule. In some embodiments, the tolerogenic factor is a siRNA that inhibits the costimulatory molecule. In some embodiments, the tolerogenic factor increases the activity of a transcriptional regulator that suppresses expression of the costimulatory molecule. In some embodiments, the tolerogenic factor increases the activity of a protein inhibitor that suppresses expression of the costimulatory molecule. In some embodiments, the tolerogenic factor comprises nucleic acid encoding a suppressor of the costimulatory molecule. In some embodiments, the tolerogenic factor degrades the costimulatory molecule. In some embodiments, the tolerogenic factor labels the costimulatory molecule for destruction. For example, the tolerogenic factor may enhance ubiquitination of the costimulatory molecule, thereby targeting it for destruction.

In some embodiments, the tolerogenic factor enhances the expression and/or activity of an immunosuppressive molecule. In some embodiments, the immunosuppressive molecule is a co-inhibitory molecule, a transcriptional regulator, or an immunosuppressive molecule. Co-inhibitory molecules negatively regulate the activation of lymphocytes. Exemplary co-inhibitory molecules include, without limitation, PD-L1, PD-L2, HVEM, B7-H3, TRAIL, immunoglobulin-like transcripts (ILT) receptors (ILT2, ILT3, ILT4), FasL, CTLA4, CD39, CD73, and B7-H4. In some embodiments, the co-inhibitory molecule is PD-L1 or PD-L2. In some embodiments, the tolerogenic factor increases the activity of the co-inhibitory molecule. In some embodiments, the tolerogenic factor increases expression of a co-inhibitory molecule. In some embodiments, the tolerogenic factor encodes the co-inhibitory molecule. In some embodiments, the tolerogenic factor increases the activity of the co-inhibitory molecule. In some embodiments, the tolerogenic factor increases the activity of a transcriptional regulator that enhances expression of the co-inhibitory molecule. In some embodiments, the tolerogenic factor increases the activity of a polypeptide that increases expression of the co-inhibitory molecule. In some embodiments, the tolerogenic factor comprises nucleic acid encoding an enhancer of the co-inhibitory molecule. In some embodiments, the tolerogenic factor inhibits an inhibitor of a co-inhibitory molecule.

In some embodiments, the tolerogenic factor increases expression and/or activity of an immunosuppressive molecule. Exemplary immunosuppressive molecules include, without limitation, arginase-1 (ARG1), indoleamine 2,3-dioxygenase (IDO), Prostaglandin E2 (PGE2), inducible nitric-oxide synthase (iNOS), nitric oxide (NO), nitric-oxide synthase 2 (NOS2), thymic stromal lymphopoietin (TSLP), vascular intestinal peptide (VIP), hepatocyte growth factor (HGF), transforming growth factor beta (TGFβ), IFNα, IL-4, IL-10, IL-13, and IL-35. In some embodiments, the immunosuppressive molecule is NO or IDO. In some embodiments, the tolerogenic factor encodes the immunosuppressive molecule. In some embodiments, the tolerogenic factor increases the activity of the immunosuppressive molecule. In some embodiments, the tolerogenic factor increases the activity of a transcriptional regulator that enhances expression of the immunosuppressive molecule. In some embodiments, the tolerogenic factor increases the activity of a polypeptide that enhances expression of the immunosuppressive molecule. In some embodiments, the tolerogenic factor comprises nucleic acid encoding an enhancer of the immunosuppressive molecule. In some embodiments, the tolerogenic factor inhibits a negative regulator of an immunosuppressive molecule.

In some embodiments, the tolerogenic factor inhibits expression and/or activity of an inflammatory molecule. In some embodiments, the inflammatory molecule is an inflammatory transcription factor. In some embodiments, the tolerogenic factor inhibits the inflammatory transcription factor. In some embodiments, the tolerogenic factor decreases expression of an inflammatory transcription factor. In some embodiments, the inflammatory transcription factor is NF-κB, an interferon regulatory factor (IRF), or a molecule associated with the JAK-STAT signaling pathway. The NF-κB pathway is a prototypical proinflammatory signaling pathway that mediates the expression of proinflammatory genes including cytokines, chemokines, and adhesion molecules. Interferon regulatory factors (IRFs) constitute a family of transcription factors that can regulate the expression of proinflammatory genes. The JAK-STAT signaling pathway transmits information from extracellular cytokine signals to the nucleus, resulting in DNA transcription and expression of genes involved in immune cell proliferation and differentiation. The JAK-STAT system consists of a cell surface receptor, Janus kinases (JAKs), and Signal Transducer and Activator of Transcription (STAT) proteins. Exemplary JAK-STAT molecules include, without limitation, JAK1, JAK2, JAK 3, Tyk2, STAT1, STAT2, STAT3, STAT4, STAT5 (STAT5A and STAT5B), and STAT6. In some embodiments, the tolerogenic factor enhances expression of a suppressor of cytokine signaling (SOCS) protein. SOCS proteins may inhibit signaling through the JAK-STAT pathway. In some embodiments, the tolerogenic factor inhibits the expression of a nucleic acid encoding the inflammatory transcription factor. In some embodiments, the tolerogenic factor deletes a nucleic acid encoding the inflammatory transcription factor. In some embodiments, the tolerogenic factor increases the activity of a transcriptional regulator that suppresses expression of the inflammatory transcription factor. In some embodiments, the tolerogenic factor increases the activity of a protein inhibitor that suppresses expression of the inflammatory transcription factor. In some embodiments, the tolerogenic factor comprises nucleic acid encoding a suppressor of the inflammatory transcription factor.

In some embodiments, the tolerogenic factor enhances expression and/or activity of an anti-inflammatory molecule. In some embodiments, the anti-inflammatory molecule is an anti-inflammatory transcription factor. In some embodiments, the tolerogenic factor enhances the anti-inflammatory transcription factor. In some embodiments, the tolerogenic factor increases expression of an anti-inflammatory transcription factor. In some embodiments, the tolerogenic factor enhances expression of nucleic acid encoding the anti-inflammatory transcription factor. In some embodiments, the tolerogenic factor decreases the activity of a transcriptional regulator that suppresses expression of the anti-inflammatory transcription factor. In some embodiments, the tolerogenic factor decreases the activity of a protein inhibitor that suppresses expression of the anti-inflammatory transcription factor. In some embodiments, the tolerogenic factor comprises nucleic acid encoding an enhancer of the anti-inflammatory transcription factor.

In some embodiments, the tolerogenic factor comprises a nucleic acid. In some embodiments, the tolerogenic factor is a nucleic acid. Exemplary nucleic acids include, without limitation, recombinant nucleic acids, DNA, recombinant DNA, cDNA, genomic DNA, RNA, siRNA, mRNA, saRNA, miRNA, lncRNA, tRNA, guide RNA, and shRNA. In some embodiments, the nucleic acid is homologous to a nucleic acid in the cell. In some embodiments, the nucleic acid is heterologous to a nucleic acid in the cell. In some embodiments, the tolerogenic factor is a plasmid. In some embodiments, the nucleic acid is a therapeutic nucleic acid. In some embodiments, the nucleic acid encodes a therapeutic polypeptide. In some embodiments, the tolerogenic factor comprises a nucleic acid encoding siRNA, mRNA, miRNA, lncRNA, tRNA, or shRNA. For example, the tolerogenic factor can include siRNA to knock down expression of inflammatory genes. In some embodiments, the tolerogenic factor is a DNA sequence that binds NFκB and prevents NFκB activation and downstream signaling.

In some embodiments, the tolerogenic factor comprises a polypeptide. In some embodiments, the tolerogenic factor is a polypeptide. In some embodiments, the protein or polypeptide is a therapeutic protein, antibody, fusion protein, antigen, synthetic protein, reporter marker, or selectable marker. In some embodiments, the protein is a gene-editing protein or nuclease such as a zinc-finger nuclease (ZFN), transcription activator-like effector nuclease (TALEN), mega nuclease, CRE recombinase, transposase, CAS9 enzyme, or integrase enzyme. In some embodiments, the fusion proteins can include, without limitation, chimeric protein drugs such as antibody drug conjugates or recombinant fusion proteins such as proteins tagged with GST or streptavidin. In some embodiments, the compound is a transcription factor. Exemplary transcription factors include, without limitation, Oct5, Sox2, c-Myc, Klf-4, T-bet, GATA3, FoxP3, and RORyt. In some embodiments, the polypeptide is IL-4, IL-10, IL-13, IL-35, IFNα, or TGFβ. In some embodiments, the polypeptide is a therapeutic polypeptide. In some embodiments, the polypeptide is a fragment of a therapeutic polypeptide. In some embodiments, the polypeptide is a peptide nucleic acid (PNA).

In some embodiments, the tolerogenic factor comprises a protein-nucleic acid complex. In some embodiments, the tolerogenic factor is a protein-nucleic acid complex. In some embodiments, protein-nucleic acid complexes, such as clustered regularly interspaced short palindromic repeats (CRISPR)-Cas9, are used in genome editing applications. These complexes contain sequence-specific DNA-binding domains in combination with nonspecific DNA cleavage nucleases. These complexes enable targeted genome editing, including adding, disrupting, or changing the sequence of a specific gene. In some embodiments, a disabled CRISPR is used to block or induce transcription of a target gene. In some embodiments, the tolerogenic factor contains a Cas9 protein and a guide RNA or donor DNA. In some embodiments, the tolerogenic factor includes a nucleic acid encoding for a Cas9 protein and a guide RNA or donor DNA. In some embodiments, the gene editing complex targets expression of a costimulatory molecule (e.g., CD80 and/or CD86).

In some embodiments, the tolerogenic factor comprises a chimeric antigen receptor (CAR). In some embodiments, the tolerogenic factor is a chimeric antigen receptor (CAR). In some embodiments, the CAR is a fusion of an extracellular recognition domain (e.g., an antigen-binding domain), a transmembrane domain, and one or more intracellular signaling domains. Upon antigen engagement, the intracellular signaling portion of the CAR can initiate an immunosuppression or tolerogenic-related response in an immune cell. In some embodiments, the CAR is a chimeric T-cell antigen receptor. In some embodiments, the CAR antigen-binding domain is a single-chain antibody variable fragment (scFv). In some embodiments, the tolerogenic factor encodes a modified TCR containing cytoplasmic signaling domains that trigger production of immunosuppressive cytokines upon binding to antigen. In some embodiments, the tolerogenic factor encodes a chimeric antigen receptor containing cytoplasmic signaling domains that trigger production of immunosuppressive cytokines upon binding to antigen.

In some embodiments, the tolerogenic factor comprises a small molecule. In some embodiments, the tolerogenic factor is a small molecule. In some embodiments, the small molecule inhibits the activity of a costimulatory molecule, enhances the activity of a co-inhibitory molecule, and/or inhibits the activity of an inflammatory molecule. Exemplary small molecules include, without limitation, pharmaceutical agents, metabolites, and radionuclides or radionuclide-containing molecules. In some embodiments, the pharmaceutical agent is a therapeutic drug and/or cytotoxic agent. In some embodiments, the compound comprises a nanoparticle. Examples of nanoparticles include gold nanoparticles, quantum dots, carbon nanotubes, nanoshells, dendrimers, and liposomes. In some embodiments, the nanoparticle contains or is linked (covalently or noncovalently) to a therapeutic molecule. In some embodiments, the nanoparticle contains a nucleic acid, such as mRNA or cDNA.

In certain aspects, the invention provides methods for delivering a tolerogenic factor into an immune cell, the method comprising passing a cell suspension comprising the immune cell through a constriction, wherein said constriction deforms the immune cell, thereby causing a perturbation of the cell such that the tolerogenic factor enters the cell, wherein said cell suspension is contacted with the tolerogenic factor. In some embodiments, the tolerogenic factor is delivered to the immune cell in vitro, ex vivo, or in vivo.

In some embodiments, the compound to deliver is purified. In some embodiments, the compound is at least about 60% by weight (dry weight) the compound of interest. In some embodiments, the purified compound is at least about 75%, 90%, or 99% the compound of interest. In some embodiments, the purified compound is at least about 90%, 91%, 92%, 93%, 94%, 95%, 98%, 99%, or 100% (w/w) the compound of interest. Purity is determined by any known methods, including, without limitation, column chromatography, thin layer chromatography, HPLC analysis, NMR, mass spectrometry, or SDS-PAGE. Purified DNA or RNA is defined as DNA or RNA that is free of exogenous nucleic acids, carbohydrates, and lipids.

In some embodiments, the compound is an intermediate compound. The intermediate compound may be a molecular entity that is formed from preceding intermediates and reacts further to give the final reaction product. In some embodiments, the intermediate compound is a protein precursor, or pro-protein, that is cleaved by an enzyme to produce the mature, functional form of the protein. In some embodiments, the intermediate compound is an inactive enzyme precursor, or zymogen, that requires modification or cleavage to produce the active enzyme.

### X. APPLICATIONS

In some aspects, the disclosure provides methods of treating a patient by introducing an immune cell, modified by passing through a constriction such that a compound enters the cell, to the patient. In some embodiments, the treatment comprises multiple (such as any of 2, 3, 4, 5, 6, or more) steps of introducing such modified immune cells to the patient. In some embodiments, the cell is isolated from a patient, modified according to the methods disclosed, and introduced back into the patient. For example, a population of immune cells is isolated from a patient, passed through the constriction to achieve delivery of a compound, and then re-infused into the patient to augment a therapeutic immune response. In some embodiments, the cell is isolated from an individual, modified according to the disclosed methods, and introduced back into the individual. For example, a population of immune cells is isolated from an individual, passed through the constriction to achieve delivery of a compound, and then re-infused into the patient to suppress an immune response or induce tolerance in the individual.

In some embodiments, the disclosure provides methods of treating an individual by introducing the cell, modified by passing through a constriction such that a compound enters the cell, to the individual. In some embodiments, the cell is an autologous cell. For example, the immune cell is isolated from an individual (e.g, a patient), modified according to the methods disclosed, and introduced back into the individual. In some embodiments, the cell is isolated from an individual, modified according to the disclosed methods, and introduced back into the same individual. In some embodiments, the cell is an allogeneic cell. For example, the cell is isolated from a different individual, modified according to the methods disclosed, and introduced into the first individual (e.g., the patient). In some embodiments, the cell is isolated from an individual, modified according to the disclosed methods, and introduced into a different individual. In some embodiments, a population of cells is isolated from an individual (the patient) or different individual, passed through the constriction to achieve delivery of a compound that induces de novo antibody production, and then infused into a patient to augment a therapeutic response.

In some embodiments, the treatment comprises multiple (such as any of 2, 3, 4, 5, 6, or more) steps of administering modified immune cells as described herein to the individual. For example, in some embodiments, there is provided a method of treating an individual by administering a cell, modified by passing through a constriction such that a compound enters the cell, to the individual 2, 3, 4, 5, 6, or more times. In some embodiments, the duration of time between any two consecutive administrations of the cell is at least about 1 day (such as at least about any of 2 days, 3 days, 4 days, 5 days, 6 days, 1 week, 2 weeks, 3weeks, 1 month, 2 months, 3 months, 4 months, 5 months, 6 months, 7 months, 8 months, 9 months, 10 months, 11 months, 1 year, or longer, including any ranges between these values).

Any of the methods described above are carried out in vitro, ex vivo, or in vivo. For in vivo applications, the device may be implanted in a vascular lumen, e.g., an in-line stent in an artery or vein. In some embodiments, the methods are used as part of a bedside system for ex-vivo treatment of patient cells and immediate reintroduction of the cells into the patient. Such methods could be employed as a means of suppressing an immune response or inducing tolerance in an individual. In some embodiments, the method can be implemented in a typical hospital laboratory with a minimally trained technician. In some embodiments, a patient operated treatment system can be used. In some embodiments, the method is implemented using an in-line blood treatment system, in which blood is directly diverted from a patient, passed through the constriction, resulting in compound delivery to blood cells, and directly transfused back into the patient after treatment.

### XI. SYSTEMS AND KITS

In some aspects, the disclosure provides a system comprising the constriction, cell suspension, and compound for use in the methods disclosed herein. The system can include any embodiment described for the methods disclosed above, including microfluidic channels or a surface having pores to provide cell-deforming constrictions, cell suspensions, cell perturbations, delivery parameters, compounds, and/or applications etc. In some embodiment, the cell-deforming constrictions are sized for delivery to immune cells. In some embodiments, the delivery parameters, such as operating flow speeds, cell and compound concentration, velocity of the cell in the constriction, and the composition of the cell suspension (e.g., osmolarity, salt concentration, serum content, cell concentration, pH, etc.) are optimized for maximum response of a compound for suppressing an immune response or inducing tolerance.

Also disclosed are kits or articles of manufacture for use in delivering a compound to suppress an immune response or induce tolerance. In some embodiments, the kits comprise the compositions described herein (e.g. a microfluidic channel or surface containing pores, cell suspensions, and/or compounds) in suitable packaging. Suitable packaging materials are known in the art, and include, for example, vials (such as sealed vials), vessels, ampules, bottles, jars, flexible packaging (e.g., sealed Mylar or plastic bags), and the like. These articles of manufacture may further be sterilized and/or sealed.

The disclosure also provides kits comprising components of the methods described herein and may further comprise instruction(s) for performing said methods to suppress an immune response or induce tolerance. The kits described herein may further include other materials, including other buffers, diluents, filters, needles, syringes, and package inserts with instructions for performing any methods described herein; e.g., instructions for suppressing an immune response or inducing tolerance.

### EXAMPLES

The following examples are given for the purpose of illustrating various embodiments of the disclosure and are not meant to limit the present disclosure in any fashion. One skilled in the art will appreciate readily that the present disclosure is well adapted to carry out the objects and obtain the ends and advantages mentioned, as well as those objects, ends and advantages inherent herein.

### Example 1: Constriction-mediated delivery of antigen and tolerogenic factors to B cells to induce tolerance

A series of experiments are undertaken to demonstrate induction of tolerance using model antigen and transgenic T-cells.

B cells are mixed with a tolerogenic factor and OVA antigen +/- a tolerogenic factor, and passed through a constriction within a microfluidic channel, or through a surface containing pores (such constriction-mediated delivery also referred to as SQZ). Pressure, temperature, and buffer composition are optimized to achieve delivery. CFSE labeled OT-I or OT-II T cells are transferred into CD45.1 mice, followed by transfer of the OVA and tolerogenic factor-loaded B cells. Mice are later challenged by i.d. injection of OVA+LPS. The draining popliteal lymph nodes are harvested to measure expansion of the OT-I or OT-II T cells.

### Example 2: Constriction-mediated delivery of antigen to B cells to induce tolerance

In this study, the ability of B cell antigen-presenting cells (APCs) containing intracellular antigen generated by constriction-mediated delivery of the antigen to induce *in vivo* antigen-dependent tolerance was evaluated. Mice were inoculated with antigen-specific T cells, tolerized by injection of the B cell APCs, and the number of the antigen-specific T cells and the levels of the inflammatory cytokine IFN-γ were determined.

### Materials and Methods

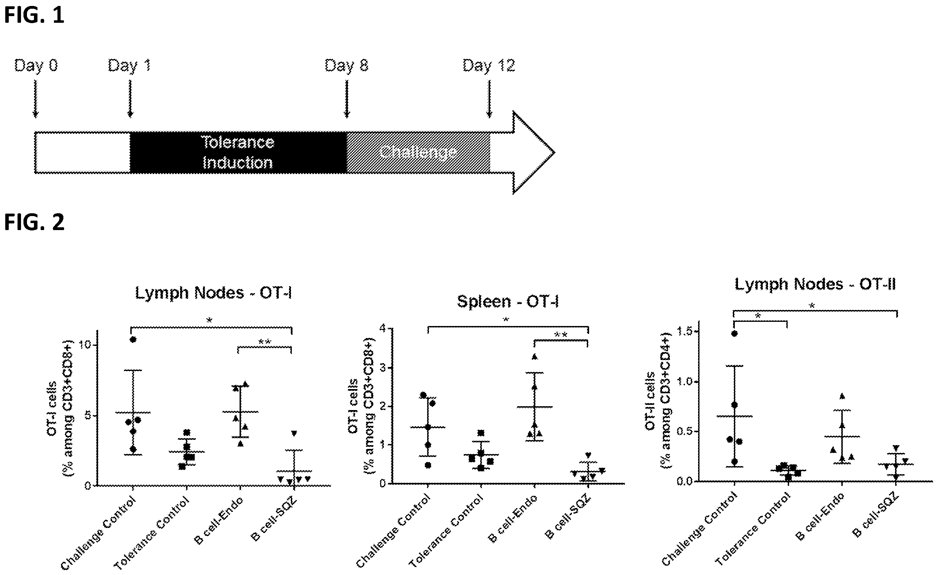

C57BL/6J recipient mice (CD45.1+) were adoptively transferred with OVA-specific CD8⁺ OT-I cells (1 x 10⁶) and CD4⁺ OT-II T cells (1 x 10⁶) (both CD45.2+) per mouse from female donor mice at day 0. On day 1, B cells from C57BL/6J mice were harvested and incubated with full OVA protein either without constriction (B cell-Endo) or with constriction-mediated intracellular delivery of the OVA protein (B cell-SQZ). 5 x 10⁶ of the respective B cells/mouse were then injected into recipient mice. Control animals received injection of either PBS (challenge control) or 10 µg free OVA (tolerance control). Antigen challenge occurred on day 8, with intradermal injection of 10 µg OVA protein + 50 ng LPS/mouse. Draining lymph nodes and spleens of each mouse were analyzed on day 12 and OT-I and OT-II T cell response to antigen challenge was measured by flow cytometry for the number of OT-I and OT-II T cells, IFN-γ intracellular staining, and ELISpot assays. A schematic of the treatment schedule is shown in FIG. 1, and the treatment groups are summarized in Table 1.

**Table 1**

| **Group** | **Day 0** | **Day 1** | **Day 8** |
|---|---|---|---|
| Challenge Control | OT-I and OT-II adoptive transfer | PBS | OVA + LPS |
| Tolerance Control | OT-I and OT-II adoptive transfer | OVA protein | OVA + LPS |
| B cell-Endo | OT-I and OT-II adoptive transfer | B cells incubated with OVA without constriction | OVA + LPS |
| B cell-SQZ | OT-I and OT-II adoptive transfer | B cells with OVA delivered by constriction | OVA + LPS |

### Results

OT-I- and OT-II-specific T cell numbers were measured on day 12 in mice from the four treatment groups. Cells from draining lymph nodes (CD3⁺/CD8⁺ gated or CD3⁺/CD4⁺ gated) and spleen (CD3⁺/CD8⁺ gated) were analyzed by flow cytometry with staining for T-cell receptor (TCR) V alpha 2 (TCRVa2) as a marker for antigen-presenting cells and CD45.2 as a marker for donor T cells. The activation and proliferation of OT-I T cells in both the draining lymph nodes and spleen was significantly inhibited (*P<0.05) in mice primed with B cells having constriction-mediated delivery of OVA protein compared to the challenge control, as indicated by the decrease in percentage of CD8+ OT-I T cells (TCRVa2+/CD45.2+) (FIG. 2, left and middle panels). This effect was dependent on constriction-mediated intracellular antigen delivery, as B cell-SQZ animals saw decreased levels of CD8+ OT-I T cells compared to mice primed with B cells incubated with OVA without constriction (***P*<0.01). The percentage of CD4+ OT-II cells (TCRVa2+/CD45.2+) was also significantly inhibited in the lymph nodes for B cell-SQZ animals compared to challenge control (FIG. 2, right panel; **P*<0.05). B cell-SQZ animals also showed a slight decrease in percentage of OT-II post-challenge compared to B cell-Endo trending towards significance, while there was no significant difference in splenic OT-II levels between treatment groups (data not shown).

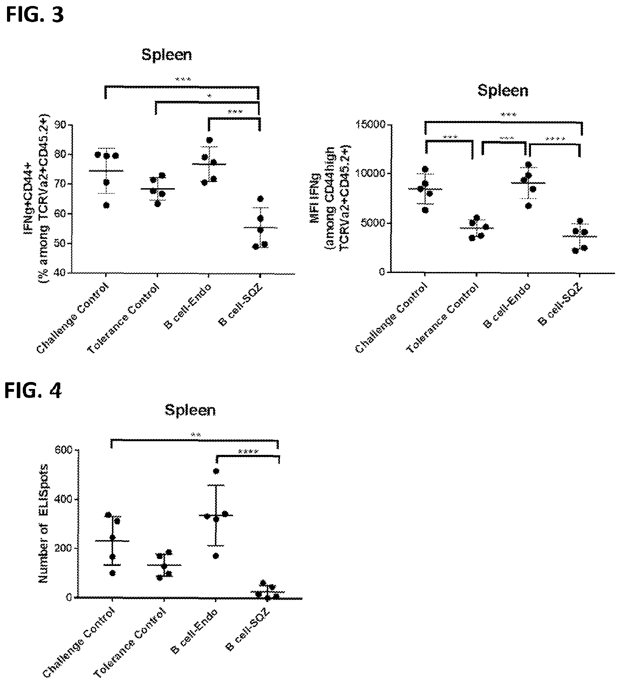

To assess the functional effect of B cell-SQZ-mediated tolerance on T cell function, the percent of splenic T cells that expressed high levels of IFN-γ (FIG. 3, left) and the level of IFN-γ production per cell (FIG. 3, right; MFI based on TCRVa2+/CD45.2+/CD44hi cells) of OT-I T cells restimulated with SIINFEKL peptide (SEQ ID NO: 1) (CD8+ active OVA epitope) were assessed for all groups by intracellular cytokine staining. OT-I T cells from B cell-SQZ mice showed decreases in both percentage of high IFN-γ expressing cells and IFN-γ production (****P*<0.0005) compared to challenge control, as well as B cell-Endo treated mice *(***P<0.0005,* *****P*<0.0001). Interestingly, B cell-SQZ mice even showed significant decreases in the percentage of high IFN-γ cells (**P*<0.05) compared to the tolerance control. The number of OT-I T cells producing IFN-γ was further confirmed by ELISpot (FIG. 4). Correlating with the flow cytometry data, B cell-SQZ animals produced a significantly lower number of IFN-γ-positive T cells compared to challenge control (***P*<0.01) and B cell-Endo (*****P*<0.0001).

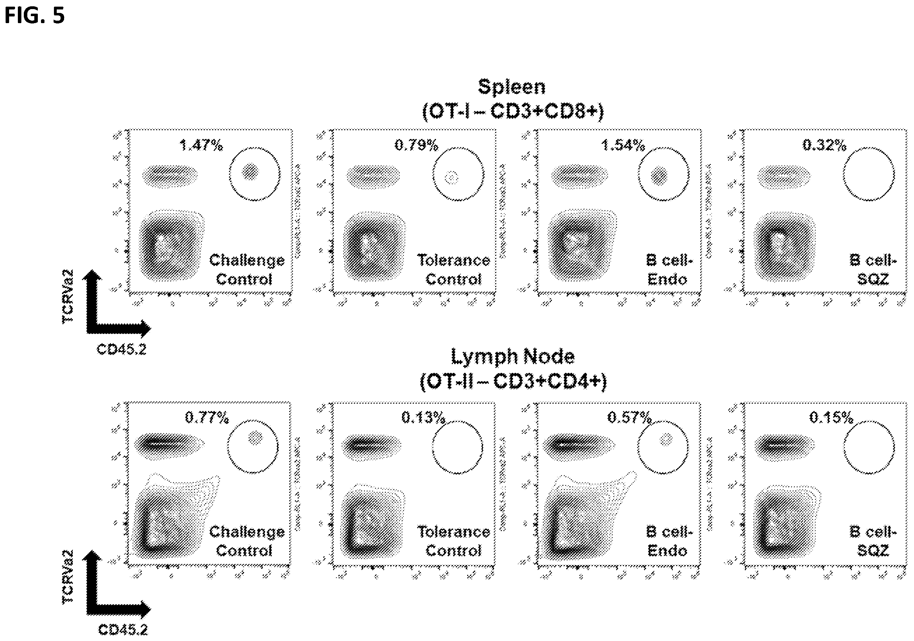

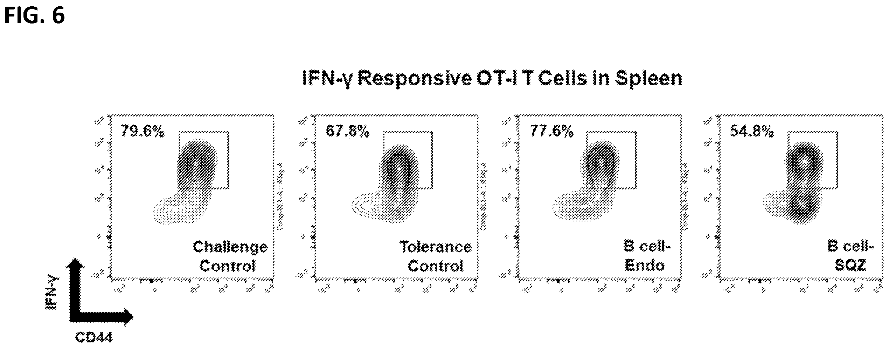

Representative flow cytograms for TCRVa2+ splenic OT-I and lymph node OT-II T cells for each treatment group show that B cell-SQZ mice have lower numbers of OT-I and OT-II compared to challenge control and B cell-Endo (FIG. 5, Table 2). Representative ICS flow cytograms for OT-I cells with high IFN-γ and CD44 antigen-activation marker show that the B cell-SQZ samples had lower numbers of IFN-γ-high T cells relative to challenge control and B cell-Endo (FIG. 6, Table 3).

**Table 2**

| | Percent OT-I cells out of CD3+/CD8+ cells | | | |
|---|---|---|---|---|
| | Challenge Control | Tolerance Control | B cell-Endo | B cell-SQZ |
| Spleen | 1.47 | 0.79 | 1.54 | 0.32 |
| Lymph Node | 0.77 | 0.13 | 0.57 | 0.15 |

**Table 3**

| Percent IFN-γ and CD44 high cells out of OT-I cells | | | |
|---|---|---|---|
| Challenge Control | Tolerance Control | B cell-Endo | B cell-SQZ |
| 79.6 | 67.8 | 77.6 | 54.8 |

### Example 3: Constriction-mediated delivery of antigen to T cells to induce tolerance

In order to determine the ability of T cell antigen-presenting cells (APCs) containing intracellular antigen generated by constriction-mediated delivery of the antigen to induce *in vivo* antigen-dependent tolerance, the number of antigen-specific T cells and the levels of inflammatory cytokine IFN-γ were measured by flow cytometry.

### Materials and Methods

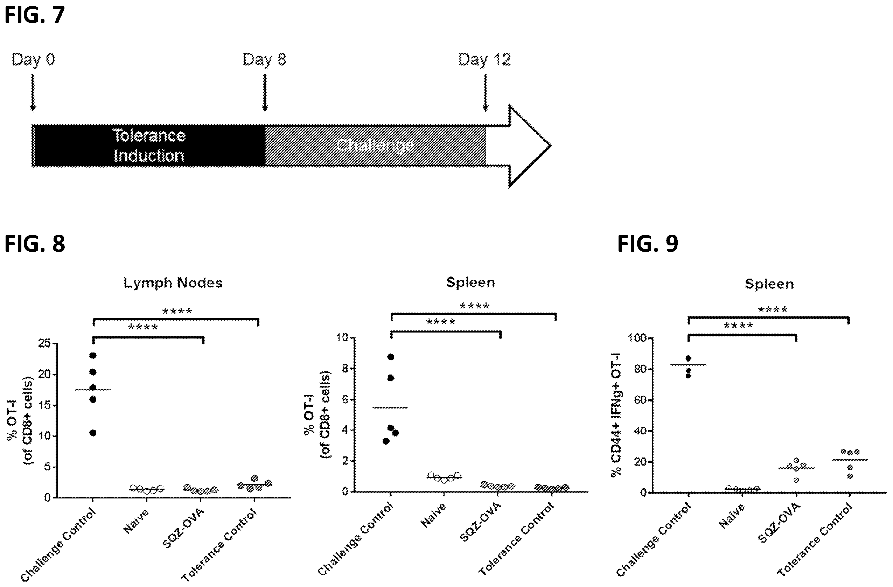

C57BL/6J recipient mice (CD45.1) were adoptively transferred with 2.5 x 10⁶ OVA-specific OT-I T cells per mouse from female OT-I donor mice (CD45.2) at day 0. On the same day, mice were also injected with 5 x 10⁶ T APC cells (CD90.1) either incubated in the presence of immunogenic OVA epitope (SIINFEKL peptide, SEQ ID NO: 1) without constriction or T cells with constriction-mediated delivery of full OVA protein. Antigen challenge occurred on day 8, with intradermal injection of 10 µg OVA protein (antigen) + 50 ng LPS (adjuvant)/mouse in challenged animals, compared to naive (no T APC cells, no antigen). OT-I T cell response to antigen challenge was measured by flow cytometry for OT-I T cell numbers and IFN-γ intracellular staining on day 12. A schematic of the treatment schedule is shown in FIG. 7, and the treatment groups are summarized in Table 4.

**Table 4**

| **Group** | **Day 0** | **Day 8** |
|---|---|---|
| Naive | OT-I adoptive transfer | |
| Challenge Control | OT-I adoptive transfer | OVA + LPS |
| Tolerance Control | OT-I adoptive transfer and T cells incubated with SIINFEKL peptide without constriction | OVA + LPS |
| T cell-SQZ | OT-I adoptive transfer and T cells with OVA delivered by constriction | OVA + LPS |

### Results

The number of OT-I-specific T cells was measured on day 12 in mice from the four treatment groups. The activation and proliferation of OT-I T cells in both the draining lymph nodes and spleen were significantly inhibited (*****P*<0.0001) in mice primed with T cells with constriction-mediated delivery of OVA protein compared to the challenge control (FIG. 8). These mice saw OT-I T cell numbers similar to naive mice or the free OVA tolerance control.

To assess the functional effect of T cell-SQZ APC-mediated tolerance on OT-I specific T cell function, the level of IFN-γ production of OT-I T cells restimulated with free SIINFEKL peptide was assessed for all groups (FIG. 9). OT-I T cells from T cell-SQZ mice showed a large decrease in the percentage of high IFN-γ expressing cells (*****P*<0.0001) compared to challenge control, similar to tolerance control (*****P*<0.0001).

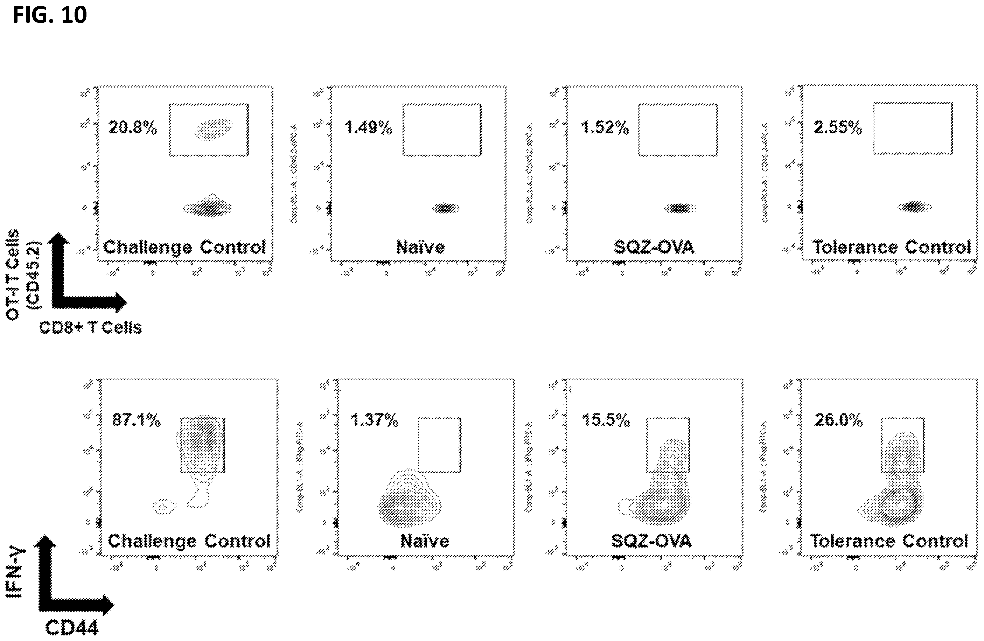

Representative flow cytograms for OT-I T cell numbers vs. CD8+ T cells (FIG. 10, top panels) and the percentage of cells with high IFN-γ vs. CD44 antigen-activation marker (FIG. 10, bottom panels) show that the T cell-SQZ animals had lower numbers for both OT-I T cells and IFN-γ production relative to challenge control (Table 5).

**Table 5**

| | Challenge Control (%) | Naive (%) | SQZ-OVA (%) | Tolerance Control (%) |
|---|---|---|---|---|
| CD45.2+/CD8+ | 20.8 | 1.49 | 1.52 | 2.55 |
| IFN-γ and CD44 high | 87.1 | 1.37 | 15.5 | 26.0 |

### Example 4A: Prophylaxis in murine type-I diabetes model

### Introduction

To determine the ability of antigen presenting cells (APCs - e.g. B and/or T cells) containing antigen delivered by SQZ induce antigen-dependent tolerance in a prophylaxis *in vivo* model of murine type-I diabetes, the levels of blood glucose is measured weekly over time after tolerance induction.

### Materials and Methods

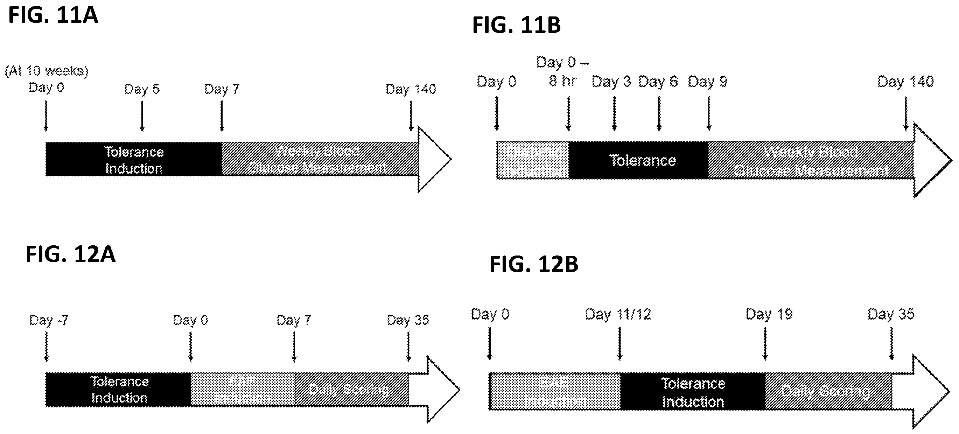

NOD/ShiltJ mice are treated with APCs at 10 weeks of age. On day 0, APCs from NOD/ShiltJ mice are harvested and incubated with insulin B-chain peptide 9-23 (InsB₉₋₂₃: CKKGSSHLVEALYLVCGERG, SEQ ID NO: 2) without constriction or InsB₉₋₂₃ is delivered by SQZ conditions, followed by injection of 5M APCs/mouse into recipient mice. Control animals receive injection with PBS (challenge control). Blood glucose measurements are carried out weekly, starting on Day 7 after treatment (age 11 weeks). Mice are considered diabetic when their blood glucose level exceeded 260 mg/dL as measured by Bayer Contour Diabetes Meter/Glucose Test Strip. A schematic of a representative treatment schedule is shown in FIG. 11A, and the treatment groups are summarized in Table 6.

**Table 6**

| **Group** | **Days 0 and 5** |
|---|---|
| Challenge control | PBS |
| APC-Endo | APCs incubated with InsB₉₋₂₃ |
| APC-SQZ | APCs SQZ'd with InsB₉₋₂₃ |

### Example 4B: Therapy in murine type-I diabetes model

### Introduction

To determine the ability of antigen presenting cells (APCs - e.g. B and/or T cells) containing antigen delivered by SQZ induce antigen-dependent tolerance in a treatment *in vivo* model of murine type-I diabetes, the levels of blood glucose is measured weekly over time after tolerance induction in mice.

### Materials and Methods

To induce rapid onset of T1D in recipients, CD4 T cells are harvested from BDC2.5 NOD mice, activated *ex vivo* with p31 mimetope peptide (YVRPLWVRME, SEQ ID NO: 3) for 4 days, and adoptively transferred (5M cells/mouse) into normoglycemic NOD recipients. Tolerance induction begins 8 hours after adoptive transfer, and is repeated every 3 days for a total of 3 doses. Recipients are treated with PBS (challenge control) or 5M APCs incubated with p31 (APC-Endo) or delivered by SQZ with p31 (APC-SQZ). Blood glucose is measured every day by Bayer Contour Diabetes Meter/Glucose Test Strip. Mice are considered diabetic when their blood glucose level exceeded 260 mg/dL. A schematic of a representative treatment schedule is shown in FIG. 11B, and the treatment groups are summarized in Table 7.

**Table 7**

| **Group** | **Days 0 and 5** |
|---|---|
| Challenge control | PBS |
| APC-Endo | APCs incubated with InsB₉₋₂₃ |
| APC-SQZ | APCs SQZ'd with InsB₉₋₂₃ |

### Example 5A: Prophylaxis in murine MS-type autoimmune disorder

### Introduction

To determine the ability of antigen presenting cells (APCs - e.g. B and/or T cells) containing antigen delivered by SQZ induce antigen-dependent tolerance in an *in vivo* prophylaxis model of a murine MS-type autoimmune disorder, the clinical score of mobility is assessed daily over time.

### Materials and Methods

Female C57BL/6 mice (10-12 wks of age) are treated with APCs prior to induction of experimental autoimmune encephalomyelitis (EAE). On days -7, APCs from C57BL/6 mice are harvested and incubated with myelin oligodendrocyte glycoprotein peptide 35-55 (MOG_{35-55:} MEVGWYRSPFSRVVHLYRNGKGS, SEQ ID NO: 4) without constriction, or MOG₃₅₋₅₅ or OVA₃₂₃₋₃₃₉ peptide (negative control: ISQAVHAAHAEINEAGRGS, SEQ ID NO: 5) are delivered to APCs by SQZ conditions, followed by injection of 5M APCs/mouse into recipient mice. On Day 0, EAE is induced by administration of MOG₃₅₋₅₅ in CFA and pertussis toxin in PBS (Hooke kit). Mice are scored daily starting on Day 7, with clinical score defined as follows: 1, limp tail; 2, partial hind leg paralysis; 3, complete hind leg paralysis; 4, complete hind and partial front leg paralysis; and 5, moribund. A schematic of a representative treatment schedule is shown in FIG. 12A, and the treatment groups are summarized in Table 8.

**Table 8**

| **Group** | **Day -7** | **Day 0 (Induction of EAE)** |
|---|---|---|
| Challenge control | APCs SQZ'd with OVA₃₂₃₋₃₃₉ | MOG₃₅₋₅₅ + pertussis toxin |
| APC-Endo | APCs incubated with MOG₃₅₋₅₅ | MOG₃₅₋₅₅ + pertussis toxin |
| APC-SQZ | APCs SQZ'd with MOG₃₅₋₅₅ | MOG₃₅₋₅₅ + pertussis toxin |

### Example 5B: Therapy in murine MS-type autoimmune disorder

### Introduction

To determine the ability of antigen presenting cells (APCs - e.g. B and/or T cells) containing antigen delivered by SQZ induce antigen-dependent tolerance in an *in vivo* model of an established murine MS-type autoimmune disorder, the clinical score of mobility is assessed daily over time.

### Materials and Methods

In female C57BL/6 mice (10-12 wks of age) experimental autoimmune encephalomyelitis (EAE) is induced by administration of MOG₃₅₋₅₅ in CFA and pertussis toxin in PBS (Hooke kit) on Day 0. Mice are then treated with APCs on the day of onset of EAE, which occurs once mice are scored ≥ 1 based on the below mobility criteria. On day ~11/12, B cells from C57BL/6 mice are harvested and incubated with myelin oligodendrocyte glycoprotein peptide 35-55 (MOG₃₅₋₅₅: MEVGWYRSPFSRVVHLYRNGKGS, SEQ ID NO: 4) without constriction, or MOG₃₅₋₅₅ or OVA₃₂₃₋₃₃₉ peptide (negative control:
ISQAVHAAHAEINEAGRGS, SEQ ID NO: 5) were delivered to APCs by SQZ conditions, followed by injection of 5M APCs/mouse into recipient mice. Mice are scored daily starting on Day 19, with clinical score defined as follows: 1, limp tail; 2, partial hind leg paralysis; 3, complete hind leg paralysis; 4, complete hind and partial front leg paralysis; and 5, moribund. A schematic of a representative treatment schedule is shown in FIG. 12B, and the treatment groups are summarized in Table 9.

**Table 9**

| **Group** | **Day -7** | **Day 0 (Induction of EAE)** |
|---|---|---|
| Challenge control | APCs SQZ'd with OVA₃₂₃₋₃₃₉ | MOG₃₅₋₅₅ + pertussis toxin |
| APC-Endo | APCs incubated with MOG₃₅₋₅₅ | MOG₃₅₋₅₅ + pertussis toxin |
| APC-SQZ | APCs SQZ'd with MOG₃₅₋₅₅ | MOG₃₅₋₅₅ + pertussis toxin |

### Sequence Listing

| SEQ ID NO | Sequence | Description |
|---|---|---|
| 1 | SIINFEKL | CD8+ active OVA epitope |
| 2 | CKKGSSHLVEALYLVCGERG | B-chain peptide 9-23 (InsB₉₋₂₃) |
| 3 | YVRPLWVRME | p31 mimetope peptide |
| 4 | MEVGWYRSPFSRVVHLYRNGKGS | myelin oligodendrocyte glycoprotein peptide 35-55 (MOG₃₅₋₅₅) |
| 5 | ISQAVHAAHAEINEAGRGS | OVA₃₂₃₋₃₃₉ peptide |

## Claims

1. A method for delivering an antigen into a tolerogenic immune cell or an immunosuppressive immune cell, the method comprising passing a cell suspension comprising the tolerogenic immune cell or the immunosuppressive immune cell through a constriction, wherein said constriction deforms the tolerogenic immune cell or the immunosuppressive immune cell, causing a perturbation of the cell such that the antigen can enter the cell, and contacting said cell suspension with the antigen,
wherein the immune cell is a B cell or a T cell,
wherein the antigen is:
(i) a polypeptide antigen;
(ii) a disease-associated antigen;
(iii) a foreign antigen;
(iv) a self-antigen;
(v) a therapeutic agent;
(vi) an allograft transplantation antigen;
(vii) a non-protein antigen;
(viii) an antigen associated with a virus; or
(ix) an adenovirus, adeno-associated virus, baculovirus, herpes virus, or retrovirus,
wherein an immune response is suppressed by at least about 10% and wherein the suppressed immune response comprises decreased production and/or secretion of one or more inflammatory cytokines,
wherein the tolerogenic immune cell or the immunosuppressive immune cell comprises intracellularly a tolerogenic factor comprising a nucleic acid encoding an arginase-1 (ARG1), a nitric-oxide synthase 2 (NOS2), an indoleamine 2,3- dioxygenase (IDO), an IL-4, an IL-10, an IL-13, a transforming growth factor-beta (TGF-β), an IL-35, or an IFN-α, and
wherein the tolerogenic immune cell or the immunosuppressive immune cell induces an immunological unresponsiveness or decreased responsiveness to challenge with the antigen.

2. The method of claim 1, wherein the tolerogenic immune cell or the immunosuppressive immune cell has not been contacted with an adjuvant.

3. The method of claim 2, wherein the adjuvant is selected from the group consisting of TLR3 and RLR ligands, TLR4 ligands, TLR5 ligands, TLR7/8 ligands, TLR9 ligands, NOD2 ligands, alum, water-in-oil emulsions, rhIL-2, anti-CD40, CD40L, IL-12, and cyclic dinucleotides.

4. The method of claim 1 or 2, wherein the tolerogenic immune cell or the immunosuppressive immune cell comprises a reduced ability to provide one or more costimulatory signals as compared to a non-tolerogenic precursor of the tolerogenic immune cell or a non-immunosuppressive precursor of the immunosuppressive immune cell.

5. The method of claim 4, wherein the one or more costimulatory signals are mediated by a molecule selected from the group consisting of CD40, CD80, CD86, CD54, CD83, CD79, and ICOS ligand.

6. The method of claim 1, 2 or 4, wherein the tolerogenic immune cell or
the immunosuppressive immune cell comprises a reduced ability to provide one or
more inflammatory signals as compared to a non-tolerogenic precursor of the tolerogenic immune cell or a non-immunosuppressive precursor of the immunosuppressive immune cell.

7. The method of claim 6, wherein the one or more inflammatory signals are mediated by a molecule selected from the group consisting of interleukin-1 (IL-1), IL-12, IL-18, tumor necrosis factor (TNF), interferon gamma (IFN-gamma), granulocyte-macrophage colony stimulating factor (GM-CSF), NF-κB, an interferon regulatory factor (IRF), and a molecule associated with the JAK-STAT signaling pathway.

8. The method of any one of claims 1, 2 or 4, wherein the antigen is presented by the tolerogenic immune cell or the immunosuppressive immune cell.

9. The method of any one of claims 1 to 8, wherein the cell suspension is contacted with the antigen before, concurrently, or after passing through the constriction.

10. The method of any one of claims 1 to 9, wherein the immune response is suppressed by at least about 15%, about 20%, about 25%, about 30%, about 40%, about 50%, about 60%, about 70%, about 75%, about 80%, about 90%, or about 100%.

11. The method of any one of claims 1 to 10, wherein the one or more inflammatory cytokines are selected from the group consisting of interleukin-1 (IL-1), IL-12, and IL-18, tumor necrosis factor (TNF), interferon gamma (IFN-gamma), and granulocyte-macrophage colony stimulating factor (GM-CSF).

12. The method of any one of claims 1 to 11, wherein the suppressed immune response comprises a decreased T cell response, an enhanced Treg response, a decreased B cell response, decreased cytokine production, a decreased autoimmune response or a decreased allergic response.

13. The method of claim 12, wherein the decreased T cell response comprises decreased T cell activation, decreased T cell survival, decreased T cell proliferation or decreased T cell functionality and wherein the decreased B cell response comprises decreased antibody production.

14. The method of any one of claims 1-13, wherein the method is repeated at least 1, 2, 3, 4, 5, or 6 times.

## Patentansprüche

1. Ein Verfahren zur Zuführung eines Antigens in eine tolerogene Immunzelle oder eine immunsupprimierende Immunzelle, wobei das Verfahren Folgendes umfasst:
Durchführen einer Zellsuspension, die die tolerogene Immunzelle oder die immunsupprimierende Immunzelle umfasst, durch eine Verengung, wobei die genannte Verengung die tolerogene Immunzelle oder die immunsupprimierende Immunzelle verformt, wodurch eine Perturbation der Zelle ausgelöst wird, sodass das Antigen in die Zelle eintreten kann, und In-Kontakt-Bringen der genannten Zellsuspension mit dem Antigen,
wobei die Immunzelle eine B-Zelle oder eine T-Zelle ist,
wobei das Antigen Folgendes ist:
(i) ein Polypeptidantigen;
(ii) ein krankheitsassoziiertes Antigen;
(iii) ein Fremdantigen;
(iv) ein Selbstantigen;
(v) ein Therapeutikum;
(vi) ein Allograft-Transplantationsantigen;
(vii) ein Nicht-Proteinantigen;
(viii)ein mit einem Virus assoziiertes Antigen; oder
(ix) ein Adenovirus, adenoassoziiertes Virus, Baculovirus, Herpesvirus oder Retrovirus,
wobei eine Immunantwort um mindestens etwa 10% supprimiert wird und wobei die supprimierte Immunantwort verminderte Produktion und/oder Sekretion von einem oder mehreren entzündlichen Zytokinen umfasst,
wobei die tolerogene Immunzelle oder die immunsupprimierende Immunzelle intrazellular einen tolerogenen Faktor umfasst, der eine Nukleinsäure umfasst, die für eine Arginase-1 (ARG1), eine Stickoxidsynthase 2 (NOS2), eine Indolamin-2,3-dioxygenase (IDO), ein IL-4, ein IL-10, ein IL-13, einen Transforming Growth Factor-beta (TGF-β), ein IL-35 oder ein IFN-α kodiert, und
wobei die tolerogene Immunzelle oder die immunsupprimierende Immunzelle eine fehlende immunologische Reagibilität oder verminderte Reagibilität auf eine Provokation mit dem Antigen induziert.

2. Verfahren nach Anspruch 1, wobei die tolerogene Immunzelle oder die immunsupprimierende Immunzelle nicht mit einem Adjuvans in Kontakt gebracht wurde.

3. Verfahren nach Anspruch 2, wobei das Adjuvans aus der Gruppe ausgewählt ist, die aus TLR3- und RLR-Liganden, TLR4-Liganden, TLR5-Liganden, TLR7/8-Liganden, TLR9-Liganden, NOD2-Liganden, Alaun, Wasser-in-Öl-Emulsionen, rhIL-2, Anti-CD40, CD40L, IL-12 und zyklischen Dinukleotiden besteht.

4. Verfahren nach Anspruch 1 oder 2, wobei die tolerogene Immunzelle oder die immunsupprimierende Immunzelle eine verringerte Fähigkeit zum Bereitstellen eines oder mehrerer kostimulatorischer Signale, verglichen mit einem nicht tolerogenen Vorläufer der tolerogenen Immunzelle oder einem nicht immunsupprimierenden Vorläufer der immunsupprimierenden Immunzelle, umfasst.

5. Verfahren nach Anspruch 4, wobei das eine oder die mehreren kostimulatorischen Signale durch ein Molekül vermittelt werden, das aus der Gruppe ausgewählt ist, die aus CD40, CD80, CD86, CD54, CD83, CD79 und ICOS-Ligand besteht.

6. Verfahren nach Anspruch 1, 2 oder 4, wobei die tolerogene Immunzelle oder die immunsupprimierende Immunzelle eine verringerte Fähigkeit zum Bereitstellen eines oder mehrerer entzündlicher Signale, verglichen mit einem nicht tolerogenen Vorläufer der tolerogenen Immunzelle oder einem nicht immunsupprimierenden Vorläufer der immunsupprimierenden Immunzelle, umfasst.

7. Verfahren nach Anspruch 6, wobei das eine oder die mehreren entzündlichen Signale durch ein Molekül vermittelt werden, das aus der Gruppe ausgewählt ist, die aus Interleukin-1 (IL-1), IL-12, IL-18, Tumornekrosefaktor (TNF), Interferon-gamma (IFN-gamma), Granulocyte-Macrophage Colony Stimulating Factor (GM-CSF), NF-κB, einem interferonregulierenden Faktor (IRF) und einem Molekül, das mit dem JAK-STAT-Signalweg assoziiert ist, besteht.

8. Verfahren nach einem der Ansprüche 1, 2 oder 4, wobei das Antigen von der tolerogenen Immunzelle oder der immunsupprimierenden Immunzelle präsentiert wird.

9. Verfahren nach einem der Ansprüche 1 bis 8, wobei die Zellsuspension vor, gleichzeitig mit oder nach dem Durchführen durch die Verengung mit dem Antigen in Kontakt gebracht wird.

10. Verfahren nach einem der Ansprüche 1 bis 9, wobei die Immunantwort um mindestens etwa 15%, etwa 20%, etwa 25%, etwa 30%, etwa 40%, etwa 50%, etwa 60%, etwa 70%, etwa 75%, etwa 80%, etwa 90% oder etwa 100% supprimiert wird.

11. Verfahren nach einem der Ansprüche 1 bis 10, wobei das eine oder die mehreren entzündlichen Zytokine aus der Gruppe ausgewählt sind, die aus Interleukin-1 (IL-1), IL-12 und IL-18, Tumornekrosefaktor (TNF), Interferon-gamma (IFN-gamma) und Granulocyte-Macrophage Colony Stimulating Factor (GM-CSF) besteht.

12. Verfahren nach einem der Ansprüche 1 bis 11, wobei die supprimierte Immunantwort eine verminderte T-Zell-Antwort, eine verstärkte Treg-Antwort, eine verminderte B-Zell-Antwort, eine verminderte Zytokinproduktion, eine verminderte Autoimmunantwort oder eine verminderte allergische Reaktion umfasst.

13. Verfahren nach Anspruch 12, wobei die verminderte T-Zell-Antwort eine verminderte T-Zell-Aktivierung, ein vermindertes T-Zell-Überleben, eine verminderte T-Zell-Proliferation oder eine verminderte T-Zell-Funktionalität umfasst und wobei die verminderte B-Zell-Antwort eine verminderte Antikörperproduktion umfasst.

14. Verfahren nach einem der Ansprüche 1-13, wobei das Verfahren mindestens 1, 2, 3, 4, 5 oder 6 Mal wiederholt wird.

## Revendications

1. Procédé d'introduction d'un antigène dans une cellule immunitaire tolérogène ou une cellule immunitaire immunosuppressive, le procédé comprenant le fait de faire passer une suspension de cellules comprenant la cellule immunitaire tolérogène ou la cellule immunitaire immunosuppressive dans une constriction, ladite constriction déformant la cellule immunitaire tolérogène ou la cellule immunitaire immunosuppressive, en provoquant une perturbation de la cellule telle que l'antigène peut pénétrer dans la cellule, et la mise en contact de ladite suspension de cellules avec l'antigène,
dans lequel la cellule immunitaire est un lymphocyte B ou un lymphocyte T,
dans lequel l'antigène est :
(i) un antigène polypeptidique ;
(ii) un antigène associé à une maladie ;
(iii) un antigène étranger ;
(iv) un autoantigène ;
(v) un agent thérapeutique ;
(vi) un antigène de transplantation d'allogreffe ;
(vii) un antigène non protéique ;
(viii)un antigène associé à un virus ; ou
(ix) un adénovirus, un virus adéno-associé, un baculovirus, un virus de l'herpès ou un rétrovirus,
dans lequel une réponse immunitaire est supprimée d'au moins environ 10 % et dans lequel la réponse immunitaire supprimée comprend une production et/ou une sécrétion diminuée(s) d'une ou plusieurs cytokines inflammatoires,
dans lequel la cellule immunitaire tolérogène ou la cellule immunitaire immunosuppressive comprend intracellulairement un facteur tolérogène comprenant un acide nucléique codant une arginase-1 (ARG1), une synthase d'oxyde nitrique 2 (NOS2), une indoleamine 2,3-dioxygénase (IDO), une IL-4, une IL-10, une IL-13, un facteur de croissance transformant bêta (TGF-β), une IL-35, ou un IFN-α, et
dans lequel la cellule immunitaire tolérogène ou la cellule immunitaire immunosuppressive induit une non-réactivité immunologique ou une réactivité réduite à une stimulation par l'antigène.

2. Procédé selon la revendication 1, dans lequel la cellule immunitaire tolérogène ou la cellule immunitaire immunosuppressive n'a pas été mise en contact avec un adjuvant.

3. Procédé selon la revendication 2, dans lequel l'adjuvant est sélectionné dans le groupe constitué des ligands TLR3 et RLR, des ligands TLR4, des ligands TLR5, des ligands TLR7/8, des ligands TLR9, des ligands NOD2, de l'alun, des émulsions eau-dans-huile, de la rhIL-2, des anti-CD40, des CD40L, de l'IL-12, et des dinucléotides cycliques.

4. Procédé selon la revendication 1 ou 2, dans lequel la cellule immunitaire tolérogène ou la cellule immunitaire immunosuppressive comporte une capacité réduite à produire un ou plusieurs signaux de costimulation comparativement à un précurseur non tolérogène de la cellule immunitaire tolérogène ou à un précurseur non immunosuppresseur de la cellule immunitaire immunosuppressive.

5. Procédé selon la revendication 4, dans lequel les un ou plusieurs signaux de costimulation sont médiés par une molécule sélectionnée dans le groupe constitué de CD40, CD80, CD86, CD54, CD83, CD79, et du ligand ICOS.

6. Procédé selon la revendication 1, 2 ou 4, dans lequel la cellule immunitaire tolérogène ou la cellule immunitaire immunosuppressive comporte une capacité réduite à produire un ou plusieurs signaux inflammatoires comparativement à un précurseur non tolérogène de la cellule immunitaire tolérogène ou à un précurseur non immunosuppresseur de la cellule immunitaire immunosuppressive.

7. Procédé selon la revendication 6, dans lequel les un ou plusieurs signaux inflammatoires sont médiés par une molécule sélectionnée dans le groupe constitué de l'interleukine-1 (IL-1), de l'IL-12, de l'IL-18, du facteur de nécrose tumorale (TNF), de l'interféron gamma (IFN-gamma), du facteur de stimulation des colonies de granulocytes et de macrophages (GM-CSF), de NF-kB, d'un facteur régulateur de l'interféron (IRF), et d'une molécule associée à la voie de signalisation JAK-STAT.

8. Procédé selon l'une quelconque des revendications 1, 2 ou 4, dans lequel l'antigène est présenté par la cellule immunitaire tolérogène ou la cellule immunitaire immunosuppressive.

9. Procédé selon l'une quelconque des revendications 1 à 8, dans lequel la suspension de cellules est mise en contact avec l'antigène avant, simultanément, ou après être passée dans la constriction.

10. Procédé selon l'une quelconque des revendications 1 à 9, dans lequel la réponse immunitaire est supprimée d'au moins environ 15 %, environ 20 %, environ 25 %, environ 30 %, environ 40 %, environ 50 %, environ 60 %, environ 70 %, environ 75 %, environ 80 %, environ 90 % ou environ 100 %.

11. Procédé selon l'une quelconque des revendications 1 à 10, dans lequel les une ou plusieurs cytokines inflammatoires sont sélectionnées dans le groupe constitué de l'interleukine-1 (IL-1), de l'IL-12 et de l'IL-18, du facteur de nécrose tumorale (TNF), de l'interféron gamma (IFN-gamma), et du facteur de stimulation des colonies de granulocytes et de macrophages (GM-CSF).

12. Procédé selon l'une quelconque des revendications 1 à 11, dans lequel la réponse immunitaire supprimée comprend une réponse diminuée des lymphocytes T, une réponse accrue de Treg, une réponse diminuée des lymphocytes B, une production diminuée de cytokines, une réponse auto-immune diminuée, ou une réponse allergique diminuée.

13. Procédé selon la revendication 12, dans lequel la réponse diminuée des lymphocytes T comprend une activation diminuée des lymphocytes T, une survie diminuée des lymphocytes T, une prolifération diminuée des lymphocytes T ou une fonctionnalité diminuée des lymphocytes T, et dans lequel la réponse diminuée des lymphocytes B comprend une production diminuée d'anticorps.

14. Procédé selon l'une quelconque des revendications 1 à 13, le procédé étant répété au moins 1, 2, 3, 4, 5 ou 6 fois.
